# EUROPÄISCHE PATENTANMELDUNG

(11) **EP 1 619 197 A1**
(43) Veröffentlichungstag der Anmeldung: **25.01.2006**
(21) Anmeldenummer: 04017543.2
(22) Anmeldetag: 23.07.2004
(51) Int. Cl.: C07D 495/14, C07D 491/14, A61K 31/519, A61P 9/10

(54) **Substituierte Pyrido[3',2':4,5]thieno[3,2-d]pyrimidin-2,4(1H,3H)dione und -4(3H)one sowie Pyrido[3',2':4,5]furo[3,2-d]-pyrimidin-2,4(1H,3H)dione und -4(3H)one zur Verwendung als Inhibitoren der TNF-alpha Freisetzung**

(71) Anmelder: Curacyte Discovery GmbH, 04103 Leipzig (DE)
(72) Erfinder: Reichelt, Claudia, 04299 Leipzig (DE); Ludwig, Alexander, 04275 Leipzig (DE); Schulze, Alexander, 04317 Leipzig (DE); Daghish, Mohammed, 04107 Leipzig (DE); Leistner, Siegfried, 04340 Leipzig (DE)
(74) Vertreter: Schüssler, Andrea

(57) **Zusammenfassung**

Die Erfindung betrifft neue Pyrido[3',2':4,5]thieno[3,2-d]pyrimidin-2,4(1H,3H)-dione und -4(3H)-one (Y=S)
sowie Pyrido[3',2':4,5]furo[3,2-d]pyrimidin-2,4(1H,3H)-dione und -4(3H)-one (Y=O) der allgemeinen Formeln 1a und 1b

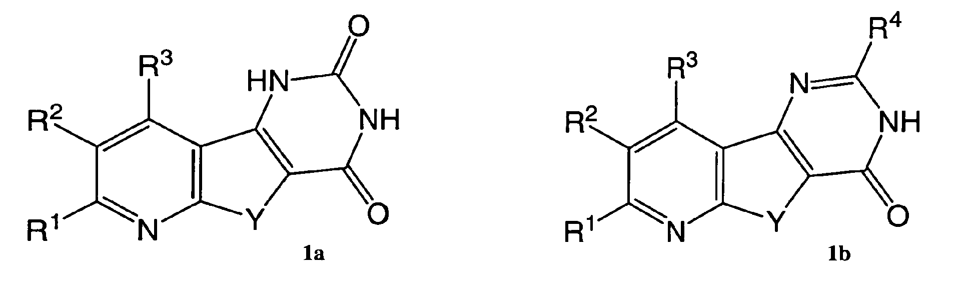

Verfahren zu deren Herstellung, pharmazeutische Zubereitungen, die diese Verbindungen und/oder deren Tautomere und daraus herstellbare physiologisch verträgliche Salze und/oder deren Solvate enthalten, sowie die pharmazeutische Verwendung dieser Verbindungen, deren Tautomere, Salze oder Solvate, als Inhibitoren der TNFα-Freisetzung.

## Beschreibung

### Technisches Gebiet

Die Erfindung betrifft neue Pyrido[3',2':4,5]thieno[3,2-d]pyrimidin-2,4(1H,3H)-dione und -4(3H)-one (Y=S)
sowie Pyrido[3',2':4,5]furo[3,2-d]pyrimidin-2,4(1H,3H)-dione und -4(3H)-one (Y=O) der allgemeinen Formeln 1a und 1b

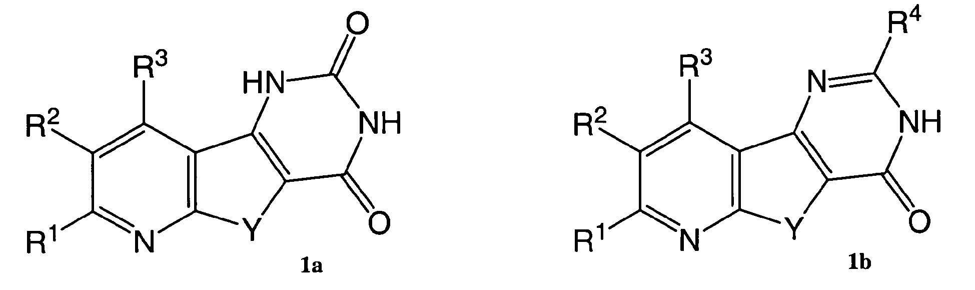

Verfahren zu deren Herstellung, pharmazeutische Zubereitungen, die diese Verbindungen und/oder deren Tautomere und daraus herstellbare physiologisch verträgliche Salze und/oder deren Solvate enthalten, sowie die pharmazeutische Verwendung dieser Verbindungen, deren Tautomere, Salze oder Solvate, als Inhibitoren der TNFα-Freisetzung.

### Stand der Technik

Die Erfindung betrifft neue Pyrido[3',2':4,5]thieno[3,2-d]pyrimidin-2,4(1H,3H)-dione und -4(3H)-one (Y=S)
sowie Pyrido[3',2':4,5]furo[3,2-d]pyrimidin-2,4(1H,3H)-dione und -4(3H)-one (Y=O) der allgemeinen Formeln 1a und 1b

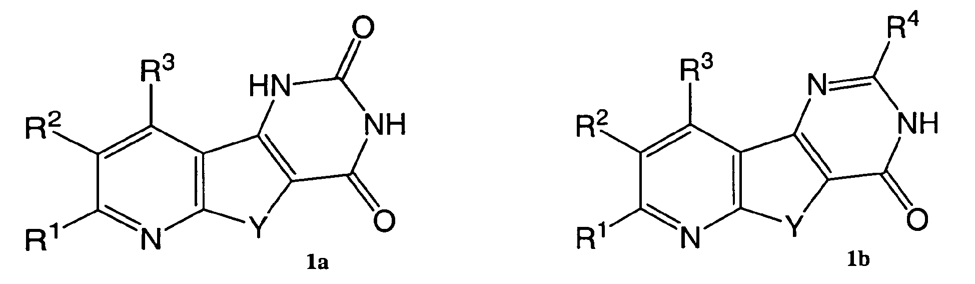

Verfahren zu deren Herstellung, pharmazeutische Zubereitungen, die diese Verbindungen und/oder deren Tautomere und daraus herstellbare physiologisch verträgliche Salze und/oder deren Solvate enthalten, sowie die Verwendung dieser Verbindungen, deren Tautomere, Salze oder Solvate als Inhibitoren der TNFα-Freisetzung, z.B. vornehmlich aus aktivierten Macrophagen, aber auch aus Mastzellen, Fibroblasten, Basophilen , Granulozyten, Endothelzellen, aktivierten Lymphozyten und Astrozyten (im Gehirn).

### Stand der Technik

Das Zytokin Tumour necrosis factor (TNFα) ist eines von heute 17 bekannten Mitgliedern einer strukturell sehr ähnlichen Proteinfamilie. Seinen Namen verdankt es der Fähigkeit, eine Nekrose von transplantierten Tumorzellen im Mausmodell zu triggern. Neben seiner Apoptose-induzierenden Wirkung wurde sehr schnell erkannt, dass TNFα auch ganz maßgeblich in die Regulation der Entzündungsantwort und der Immunantwort eingebunden ist. Eine Überproduktion von TNFα oder die Aktivierung der TNFα-vermittelten Signalkaskaden spielen in der Pathogenese einer Vielzahl von Erkrankungen, wie z.B. Sepsis, cerebrale Form der Malaria, neurodegenarativen Erkrankungen wie z.B. Mb. Alzheimer, Mb. Parkinson, bei Diabetes mellitus, COPD/Asthma, Tumorerkrankungen und hier insbesondere Tumoren des blutbildenden Systems wie z.B. Leukämien und Lymphome, virale Erkrankungen und hier insbesondere retrovirale Erkrankungen wie z.B. das erworbene Immundefizienz Syndrom (AIDS), Guillain-Barre Syndrom, Rhinitis allergica, allergische Konjunktivitis, systemische Sklerodermie, Graft versus host disease (GvHD), Systemischer Lupus Erythematodes (SLE), Osteoporosis, Toxisches Schocksyndrom, Akute Glomerulonephritis, akute und chronische Schmerzen, Arteriosklerose, Herzinfarkt, Schlaganfall, bei Sarkoidose, Multiple Sklerose, Rheumatoid Arthritis (RA), Osteoarthritis, Colitis ulcerosa, Vasculitis, Uveitis, Mb. Crohn, Mb. Behcet, Myastenia Gravis und chronisch entzündlichen Hauterkrankungen wie Psoriasis, atopische Dermatitis, Ekzeme und Alopecie, eine zentrale Rolle (*Chen G, Goeddel DV (2002 TNF-R1 signaling: a beautiful pathway. Science 296 1634-1635; Ware CF (2003) The TNF superfamily. Cytokine & Growth Factor reviews 14 181-184; Dempsey PW (2003) The signaling adaptors and pathways activated by TNF superfamily. Cytokine & Growth Factor reviews 14 193-209).*

Bei TNFα handelt es sich um eines der wichtigsten pro-inflammatorischen Zytokine, der in die Pathogenese fast aller chronisch entzündlichen Erkrankungen maßgeblich eingebunden ist. TNFα, welches auch als Chachektin, Makrophagen-Cytotoxin (MCT), E tumor necrosis factor-a und als macrophage cytotoxic factor (MCF) beschrieben wurde, wird von verschiedensten Zellen nach Stimulation mit Lipopolysaccharid (LPS), Interferronen (IFN's), IL-2, Bradykinin, GM-CSF, Antigen-Antikörper-Komplexen, Substanz P und zahlreichen weiteren biologisch aktiven Verbindungen synthetisiert und sezerniert. TNFα wird unter physiologischen Bedingungen hauptsächlich von Makrophagen, T-Lymphozyten, Mikrogliazellen und NL-Zellen gebildet. Stimulierte und somit aktivierte Fibroblasten, glatte Muskelzellen, Astrozyten, Keratinozyten, Endothelzellen und Lungen-Epithelzellen sezernieren gleichfalls TNFα.

Humanes TNFα ist ein 17 kDa großes Protein, welches aus 157 Aminosäuren besteht und zu Dimeren und Trimeren assoziiert. Es existiert eine weitere höhermolekulare Variante dieses Moleküls mit einer Molmasse von 26 kDa, das als Transmembranprotein in der Zellmembran verankert ist. Man geht heute davon aus, dass zunächst die höhermolekulare Transmembranform synthetisiert wird, deren extrazelluläre Domäne durch das TNFα converting enzyme (TACE) abgespalten wird. Das lösliche TNFα zirkuliert als ein Homotrimer und bindet sich an seine spezifischen Rezeptoren an Zelloberflächen. Die Bindung von TNFα an seine Rezeptoren (TNFR1, TNFR2) bewirkt bei diesen eine konformative Änderung und Dimerisierung bzw. Clusterung der Rezeptoren, welche über eine Signalkaskade den biologischen Effekt von TNFα vermitteln. In zahlreichen Untersuchungen konnte gezeigt werden, dass über die Bindung von TNFα an den TNFR1 die meisten biologischen Effekte realisiert werden. Dies beinhaltet die Induktion der Apoptose über eine Aktivierung der Caspase 8 und nachfolgender Aktivierung der Caspasen 3,6 und 7, die dann unmittelbar zur Apoptose der Zelle führen.

Ein weiterer wichtiger Signalweg durch TNFα ist die Aktivierung von zwei wichtigen Transkriptionsfaktoren, dem nuclear factor-kappaB (NF-_{κ}B) und c-Jun. Diese beiden Transkriptionsfaktoren spielen eine außerordentlich wichtige Rolle in der Regulation der Genexpression bei der Zelldifferenzierung, dem Zellwachstum, bei der Immun- und Entzündungsantwort, bei Zellstressregulationsvorgängen und bei der Tumorgenese. NF-_{K}B reguliert unter anderem die Gene für IL-1α, IL-1β, IL-2, IL-3, IL-6, IL-8, IL-12, TNFα, LT-a, IFN.a/β, G-CSF, M-CSF, GM-CSF, für den Zytokinrezeptor IL-2Rα, für die Adhäsionsmoleküle ICAM-1, VCAM-1, MAdCAM, E-Selektin, für die immunregulatorischen Moleküle leichte Kette des lgy, MHC Class I und II, TCRα und β, β₂ Mikroglobulin, TAP1, iNOS und für die Akute Phase Proteine SAA, α₁-saures Glycoprotein und TSG-14/PTX3.

Über die tatsächliche physiologische Bedeutung der Bindung von TNFα an den TNFR2 existieren heute noch erhebliche Widersprüche. Deshalb ist die Aufdeckung der genauen molekularen Signaltransduktionsabläufe noch Gegenstand der Grundlagenforschung. Mehrheitlich geht man heute davon aus, dass die Bindung von TNFα an den TNFR2 auch die Mitogen-aktivierten Proteinkinase Kinasen (MAPKK) aktiviert, im speziellen die MEKK1 und die ASK1, die über eine Aktivierungskaskade zur Aktivierung der c-Jun Kinase (JNK) und damit zu einer Aktivierung des Transkriptionsfaktors c-Jun führt. In diesen Regulationsweg ist auch die Aktivierung der p38 Kinase eingebunden, die zur Aktivierung von p38 führt. Die Aktivierung von p38 ist essentiell für die Produktion der pro-inflammatorischen Zytokine IL-1β, TNFα und IL-6 und ist darüber hinaus auch verantwortlich für die Induktion und Expression der mit chronischen Entzündung vergesellschafteten Enzyme COX-2 und iNOS (*Ono K, Han J (2000) The p38 signal transduction pathway: activation and function. Cell Signal 12 1-13*). Über weitere Aktivierungswege werden auch die wichtigen Transkriptionsfaktoren activatingtranscription factor 2 (ATF2) und das Aktivatorprotein-1 (AP-1) induziert, welche unmittelbar stimulierenden Einfluss auf die Expression pro-inflammatorischer Moleküle wie E-Selectin, RANTES, IL-12, IL-6 und IL-8 ausüben (*Guicciardi ME, Gores GJ (2003)J Clin Invest 111 1813-1815*).

Die erste biologische Bedeutung von TNF erkannten 1969 GRANGER et al. (*Granger GA, Shacks SJ, Williams TW, Kolb WP (1969) Lymphocyte in vitro cytotoxicity: specific release of lymphotoxin-like materials from tuberculin-sensitive lymphoid cells. Nature 221 1155-1157*) die zeigen konnten, dass ein von Lymphozyten und Makrophagen sezerniertes Protein (Lymphotoxin) zur Lyse von Zellen, insbesondere von Tumorzellen, führt. 1984 konnten GRAY et al. (*Gray PW, Aggarwal BB, Benton CV, Brüngman TS, Henzel WJ, Jarrett JA, Leung DW, Maffatt B, Ng P, Svedersky LP et al. (1984) Cloning and expression of cDNA for human lymphotoxin, a lymphokine with tumor necrosis activity. Nature 312 721-724*) und PENNICA et al. (*Pennica D, Nedwin GE, Hayflick JS, Seeburg PH, Deyrynck R, Palladino MA, Kohr WJ, Aggarwal BB, Goeddel DV (1984) Human tumor necrosis factor: precursor structure, expression and homology to lymphotoxin. Natur 312 724-729*) die cDNA für TNFα klonieren und das Protein exprimieren.

Die biologische Aktivität von TNFα wird hauptsächlich über zwei spezifische Rezeptortypen (TNFR1, TNFR2) vermittelt, die sich transmembran und mit einem extra- und intrazellulären Anteil auf einer Vielzahl Zellen des menschlichen Körpers befinden.

TNFα besitzt ein sehr breites Spektrum an biologischen Aktivitäten und reguliert fast alle Zellen. Er ist aus heutiger Sicht ein wesentlicher Mediator bei Entzündungs- und Immunreaktionen, aber auch bei der Apoptose, der Zelldifferenzierung, bei der Induktion von Fieber und zahlreichen weiteren pathophysiologischen Regulationsprozessen.

Eine zentrale Stellung nimmt TNFα bei der Endothelzellaktivierung während des Entzündungsprozesses ein. Hierbei stellt die Aktivierung der vaskulären Endothelzellen einen wesentlichen Schritt in der Initiationsphase der entzündlichen Reaktionen im Gewebe dar. So führen pro-inflammatorische Zytokine, mit TNFα an der Spitze, zur Expression endothelialer Adhäsionsmoleküle und chemotaktisch wirksamer Chemokine, die ihrerseits Makrophagen und T-Lymphozyten die Möglichkeit geben, am Endothel anzudocken und über eine aktive Wanderung ins entzündliche Gewebe (Extravasion) zu kommen. Man unterscheidet heute in diesem Zusammenhang eine lokale Wirkung von TNFα von einer systemischen. Die lokalen Effekte sind wie oben angeführt eine verstärkte Diapetese von Immun- und Entzündungszellen ins entzündliche Gewebe und eine starke Adhäsion von Thrombozyten an den Blutgefäßwänden. Der systemische Effekt von TNFα führt zu Ödemen, einer Verringerung des Blutvolumens, Hypoproteinämie, verbreitete intravaskuläre Blutgerinnung und in ihrer Maximalvariante zu multiplem Organversagen (septischer Schock).

TNFα bewirkt also eine lokale Aktivierung des vaskulären Endothels, eine Freisetzung von Stickoxid (NO) mit nachfolgender Steigerung der vaskulären Permeabilität, eine erhöhte Expression von Adhäsionsmolekülen und eine erhöhte Expression von "class II major histocompatibility molecules" (MHC II). Das Ergebnis ist ein Einwandern von Entzündungs- und Immunzellen, Antikörpern und Komplementfaktoren in das entzündliche Gewebe. TNFα verursacht gleichfalls in den lokalen Lymphknoten eine antigenspezifische Aktivierung der B- und T-Lymphozyten. Des Weiteren aktiviert TNFα Thrombozyten und verstärkt deren Adhäsion an den Gefäßwänden.

TNFα selbst induziert die Synthese anderer pro-inflammatorischer Zytokine wie IL-1, IL-6, IL-8 und GM-CSF und führt dadurch zu einem Circulus vitiosus des entzündlichen Prozesses. Zusätzlich ist TNFα noch maßgeblich in weitere pathophysiologische Prozesse, wie die Gelenkknorpelzerstörung bei rheumatischen Erkrankungen, Knochenresorptionsprozesse, Hemmung der Knochenbildung, Hemmung der Proteoglycansynthese und Induktion von Matrix Metalloproteinasen (MMP's) und Prostaglandin E₂ *(Mease P (2002) Psoriatic arthritis: The role of TNF inhibition and the effect of its inhibition with etanercept. Clin Exp Rheumatol 20 (Suppl. 28) S116-S121*) involviert.

### Übersicht der abgesicherten biologischen Wirkung von TNFα auf humane Zellen/Organe

| **Zellart/Organe** | **Biologische Wirkung** |
|---|---|
| | |
| Endothelzelle | Zytokin-Freisetzung, Expression von Adhäsionsmolekülen, Freisetzung von Gerinnungsfaktoren, Induktion der induzierbaren Stickoxid Synthase (iNOS) |
| B-Lymphozyt | Verstärkung der Antikörperproduktion |
| T-Lymphozyt | T-Zell Aktivierung, Apoptose, Freisetzung von IFNγ, IL-2 |
| Gehirn | Induktion von Fieber und Schlaf |
| Leber | Freisetzung von "Akute Phase" Proteinen |
| Adipozyt | Hemmung der Lipoprotein-Lipase, Vermittlung der Insulin Resistenz |
| Fibroblast | Zellteilung, Induktion von MMP's und Zytokinen |
| Myozyt | Induktion der Apoptose, Funktionsstörungen |
| Osteoclast | Knochenresorption |
| Macrophage | Freisetzung von TNFα nach Stimulation durch Bakterien, Viren und Zytokine |
| Monozyt | Monozyten Aktivierung, Freisetzung von TNFα, IL-1, IL-6, iNOS |
| Thrombozyten | verstärkte Thrombozytenaggregation |

### Entwicklung von TNFα Inhibitoren

In der Vergangenheit gab es zahlreiche therapeutische Strategien, um die biologische Aktivität von TNFα zu hemmen und damit den chronischen Entzündungsprozess zu unterbrechen.
- An erster Stelle standen Bemühungen, die Synthese von TNFα zu hemmen. Hierzu kamen anti-inflammatorische Zytokine, wie z.B. das IL-10, Pentoxifylline, Thalidomid bzw.-Analoga, Kortikosteroide, Cyclosporin A, PDE-4 Inhibitoren und Antisense Oligonukleotide zum Einsatz.
   Am erfolgreichsten werden seit Jahren Kortikosteroide in der akuten Phase eines schweren entzündlichen Prozesses eingesetzt. Ihre breitere und besonders längere Anwendung ist auf Grund der schweren unerwünschten Arzneimittelwirkungen stark limitiert. Diese Gründe treffen auch für das gleichfalls seit Jahren zugelassene Immunsuppresivum Cyclosporin A zu. Pentoxifylline und Thalidomidanaloga zeigten in den klinischen Studien nur eine unzureichende therapeutische Wirksamkeit.
   Der Einsatz von PDE-4 Inhibitoren zeigt über die intrazelluläre Steigerung der cAMP Konzentration einen inhibierenden Einfluss auf die TNFα Freisetzung. Momentan sind mit Cilomilast, AWD 12-81 (GSK) und Roflumilast (Altana) drei Entwicklungskandidaten in der fortgeschrittenen klinischen Prüfung bzw. stehen kurz vor der Zulassung. Die klinische Anwendung dieser Substanzen geht jedoch auch mit unerwünschten Arzneimittelwirkungen, hauptsächlich emetischer Natur, einher. Die Antisense-Therapie befindet sich noch in einer sehr frühen Entwicklungsphase und hat zumindest in den ersten Tieruntersuchungen noch nicht die erhoffte Wirksamkeit nachweisen können.
- Ein weiterer Ansatz bestand in der Inhibierung des TNFα Prozessings durch Inhibitoren der Metalloproteinase TNF converting enzyme (TACE).
   Die Entwicklung von niedermolekularen TACE Inhibitoren befindet sich noch in der Phase der angewandten Grundlagenforschung. So beschrieben TSUKIDA et al. 2004 Hydroxamsäurederivate, die TACE in vitro hemmen *(Tsukida T, Moriyama H, Inoue Y, Kondo H, Yoshino K, Nishimura S (2004) Synthesis and biological activity of selective azasugar-based TACE inhibitors. Bioorg Med Chem Lett. 22 1569-1572*). Einige Matrix Metalloproteinase Inhibitoren hemmen auch unspezifisch TACE, sind aber auf Grund ihrer MMPinhibitorischen Aktivität für eine pharmazeutische Entwicklung nicht geeignet. WILLIAMS et al. konnten überraschend zeigen, dass der MMP Inhibitor BB-2275 paradoxerweise sowohl TACE, als auch das Shedding der TNFα Rezeptoren (TNFR1, TNFR2) hemmt und dadurch keinen TNFα inhibierenden Effekt hatte (*Williams LM, Gibbons DL, Gearing A, et al. (1996) Paradoxical effects of a synthetic metalloproteinase inhibitor that blocks both p55 and p75 TNF receptor shedding and TNF alpha processing in RA synovial membrane cell cultures. J Clin Invest 97 2833-2841*). Alle heute bekannten TACE Inhibitoren sind in ihrer hemmenden Wirkung unspezifisch, d.h. auch andere wichtige Metalloenzyme werden durch sie gehemmt, mit der Wahrscheinlichkeit unerwünschter (Neben)Wirkungen.
- In neuerer Zeit haben sich Strategien zur Blockierung des TNFα durch Antikörper gegen TNFα und lösliche TNF-Rezeptoren durchgesetzt.
   Zum jetzigen Zeitpunkt sind mit Remicade® und Humira™ zwei monoklonale anti-TNF Antikörper von der FDA und auch von der EMEA als anti-inflammatorische Therapeutika zugelassen worden.
   Remicade (Essex/Centrocor) wurde durch die FDA 1998 für die Indikation Mb. Crohn und in 2000 für die Indikation Rheumatoide Arthritis zugelassen. Momentan laufen klinische Studien für die Anwendung bei Psoriasis vulgaris und Psoriasis arthropatica. Bei Remicade handelt es sich um einen chimaeren monoklonalen Antikörper gegen das humane TNFα. In den klinischen Studien zeigte das Präparat gute bis sehr gute Wirkeigenschaften beim Mb. Crohn. Jedoch wurde über teilweise erhebliche Nebenwirkungen, wie erhöhte Infektionsgefahr, Magen-Darm Beschwerden, Kopfschmerz und allergische Reaktionen berichtet. Ein Teil der Nebenwirkungen wird auf den Maus-Anteil des monoklonalen Antikörpers zurückgeführt, der vom menschlichen Organismus als "fremd" erkannt wird, wodurch Antikörper dagegen gebildet werden. Remicade wird intravenös verabreicht und die jährlichen Medikamentenkosten belaufen sich auf über $ 12.000 pro Patient.
   Humira (Abbott) ist für die Behandlung der Rheumatoiden Arthritis seit 2002 in USA und seit 2003 in Europa zugelassen. Klinische Studien zur Behandlung der Psoriasis vulgaris zeigten sehr gute Therapieerfolge. Als häufige Nebenwirkungen wurden Kopfschmerz, erhöhte Infektanfälligkeit, Magen-Darm Beschwerden und allergische Reaktionen beobachtet. Bei Humira handelt es sich um einen vollhumanisierten monoklonalen Antikörper gegen humanes TNFα. Das Präparat wird subcutan (s.c.) verabreicht. Die jährlichen Behandlungskosten belaufen sich auch bei diesem Präparat auf über $ 12.000 pro Patient.
   Enbrel (Immunex/Wyeth) wurde erstmals durch die FDA 1998 für die Indikation Rheumatoide Arthritis zugelassen und seit 2000 ist das Präparat auch auf dem europäischen Markt. Die Zulassung für die Indikation Psoriasis vulgaris und Psoriasis arthropatica wird noch 2004 von der FDA erwartet. Bei Enbrel handelt es sich um ein rekombinantes (CHO-Zellen) dimeres Fusionsprotein, bei dem zwei extrazelluläre Bindungsdomänen des p75-Anteils des TNF Rezeptors an den Fc-Anteil des humanen IgG1-Moleküls angekoppelt sind und dadurch lösliches TNFα im Blut/Gewebe abbinden und somit neutralisieren kann. Laut Herstellerangaben besitzt dieses Fusionsprotein ein geringes immunogenes Potential, wohingegen aber auch Fälle beschrieben wurden, in denen eine Antikörperbildung gegen das Fusionsprotein beobachtet wurde. Als häufige Nebenwirkungen wurden allergische Reaktionen, Infektanfälligkeit und die Bildung von Auto-Antikörpern (ANA) beschrieben. Das Präparat wird subcutan verabreicht und die jährlichen Behandlungskosten belaufen sich gleichfalls auf über $ 10.000 pro Patient.

Zusammenfassend kann festgestellt werden, dass sich das therapeutische Konzept der Hemmung von TNFα als biologischer Endpunkt, als tragfähig in der Klinik erwiesen hat. Momentan stehen nur proteinogene Präparate (monoklonale Antikörper, Fusionsproteine) zur Verfügung, die eine Reihe von unerwünschten Arzneimittelwirkungen aufweisen. Darüber hinaus ist deren intravenöse bzw. subkutane Applikationsform für Patienten sehr belastend und geht mit einer entsprechend schlechten Compliance einher. Die sehr hohen Herstellungs- und somit auch Behandlungskosten limitieren ebenfalls deren Einsatz.

Es besteht deshalb nach wie vor das dringende Bedürfnis nach niedermolekularen, nichtproteinogenen Wirkstoffen, die oral verabreicht werden können und eine bessere Verträglichkeit aufweisen. Zurzeit existieren noch keine bekannten niedermolekularen Verbindungen, die selektiv die Synthese von TNFα hemmen, ohne mit anderen Stoffwechselwegen zu interagieren.

Es besteht deshalb die dringende Aufgabe, neuartige niedermolekulare Wirkstoffe zu entwickeln, die zu einer signifikanten Inhibition der TNFα-Freisetzung aus relevanten humanen Zellpopulationen führen.

Diese Aufgabe wird von den Pyrido[3',2':4,5]thieno[3,2-d]pyrimidin-2,4(1H,3H)-dionen und -4(3H)-onen sowie Pyrido[3',2':4,5]furo[3,2-d]pyrimidin-2,4(1H,3H)-dionen und -4(3H)-onen der allgemeinen Formeln 1a und 1b 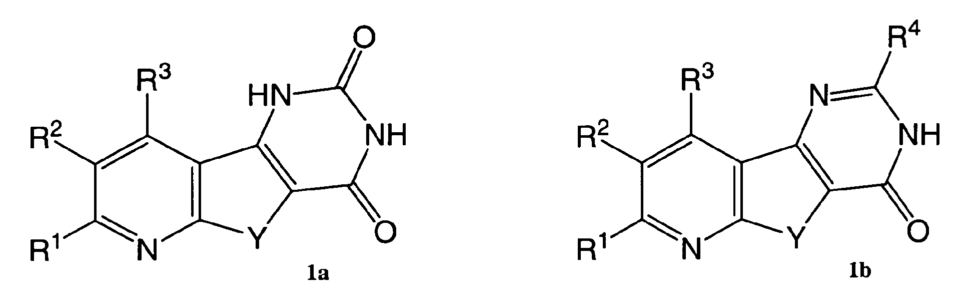 gelöst.
In bisher nur wenigen Fällen ist über biologisch aktive Pyrido[3',2':4,5]thieno-[3,2-d]pyrimidin-Derivate, jedoch noch nicht über entsprechende -2,4(1H,3H)-dione berichtet worden:

So wurden von *J.M. Quintela et al.: Bioorg. Med. 6 (1998) 1911-1925,* die Synthese von mehreren 2-Dimethylamino-(oder 2-H)-4-sek.amino-7-ethoxy-8-cyano-(oder 8-H)-9-phenyl- und auch das strukturanaloge 4-Ethoxy-pyrido[3',2':4,5]thieno-[3,2-d]pyrimidin-Derivat beschrieben. Einige dieser Verbindungen zeigten eine die Histamin-Freisetzung aus Mastzellen von Ratten inhibierende Wirkung. *Abdel-Rahmann et al.: Pharmazie 58 (2003) 372*-*377* berichteten über die Synthese von 8-Acetyl-3-amino-7-methyl-4-imino-9-subst.phenyl-pyrido[3',2':4,5]thieno-[3,2-d]pyrimidinen mit antimikrobieller Wirksamkeit. Weiterhin wurden Derivate dieses Heterosystems mit unter anderem signifikanter cholesterolsenkender (*C.J.Shishoo, M.B.Devani und V.S.Bhadti: Indian Patent 151.456 (1983); Chem. Abstr.: 100, 209858 (1984);* und *V.P.Arya, Drugs Future, 10, 123 (1985)), mit analgetischer (C.G.Dave et al.: J. Indian Chem.Soc. 66, 48 (1989))*,mit antipyretischer (*E.Bousquet et al.: Farmaco Ed.Sci. 40, 869 (1985)*; *und E. Bousquet et al.: Farmaco Ed.Sci. 39, 110 (1984)),* mit antianaphylaktischer *(H. Vieweg, S.Leistner, G. Wagner* et al.: *East German Patent DD 257830 (1988); Chem. Abstr.: 110, 95262p (1989); und H. Vieweg, S.Leistner, G. Wagner et al.: East German Patent DD 258234 (1988));* mit antiinflammatorischer (*E.F.Elslager, P.W.Jacob* und *M.Leslic: J.Heterocyclic Chem. 9, 775 (1972);* und *M.Chaykovsky* et al.: *J.Med.Chem. 10, 188 (1973);* und *L.A.Radinovskaya* und *A.Sharanin: Khim.Geterotsikl.Soedin. 805 (1988);* und *S.Leistner* et al.: *Pharmazie 41, 54 (1986));* mit klinisch effektiver antiallergischer (*G.D.Madding* und *M.D. Thompson: J.Heterocyclic Chem. 24, 581 (1987));* und mit potentiell antineoplastischer Wirksamkeit *(C.C.Cheng:in Progress in Medicinal Chemistry 25, 35 (1989)* beschrieben.

Von *M.A.A.EI-Neairy (Phosphorys, Sulfur and Silicon 1999, 189)* wurden 8-Acetyl-7-methyl-9-p-nitrophenyl-pyrido[3',2':4,5]thieno-[3,2-d]pyrimidin-2,4(1H,3H)-dion und dessen 9-p-Dimethylamino-Analogon und von *V.A.Artemov et al. (Chemistry of Heterocyclic Compounds 30, 110 (1994)* wurde das entsprechende 7,9-Dimethyl-2,4-dion-Derivat beschrieben. Angaben über pharmakologische Eigenschaften dieser Pyrido[3',2':4,5]thieno-[3,2-d]pyrimidin-2,4(1H,3H)-dione sind bisher nicht bekannt.

Als Inhibitoren der TNFα-Freisetzung sind Pyrido[3',2':4,5]thieno[3,2-d]pyrimidin-2,4(1H,3H)-dione und -4(3H)-one sowie Pyrido[3',2':4,5]furo[3,2-d]pyrimidin-2,4(1H,3H)-dione und -4(3H)-one der allgemeinen Formeln 1a und 1b jedoch bisher völlig unbekannt.

### Beschreibung der Erfindung

Die Erfindung betrifft neue Pyrido[3',2':4,5]thieno[3,2-d]pyrimidin-2,4(1H,3H)-dione und -4(3H)-one sowie
Pyrido[3',2':4,5]furo[3,2-d]pyrimidin-2,4(1H,3H)-dione und -4(3H)-one der allgemeinen Formeln 1a und 1b 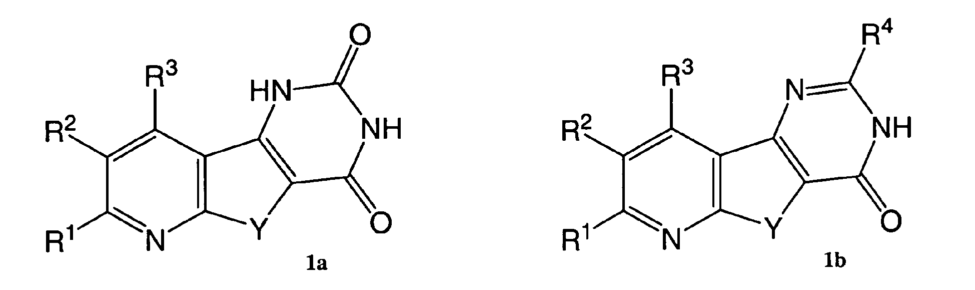
worin bedeuten:
- Y: Schwefel oder Sauerstoff und
- R¹ und R³,: gleich oder ungleich unabhängig voneinander,
- Wasserstoff,
- C₁₋₁₂Alkyl (ggf. mit R^{§} substituiert),
- C₂₋₁₂ Alkenyl und C₂₋₁₂ Alkinyl (jeweils ggf. mit R^{§} substituiert),
- Benzyl, Phenyl-(C₂₋₆)alkyl , Phenyl, 4-R^{§} -Phenyl, 3-R^{§} -Phenyl, 2-R^{§-}Phenyl, 3-R^{§},4-R^{§}-Phenyl, 3-R^{§},4-R^{§}-,5-R^{§}-Phenyl,
- Difluormethyl, Trifluormethyl,
- 1-Naphthyl, 2-Naphthyl (jeweils ggf. mit R^{§} substituiert),
- C₃₋₁₄Cydoalkyl, C₃₋₁₄Cydoalkenyl (jeweils ggf. mit R^{§}-substituiert),
- mono- oder bicyclische gesättigte oder ein- oder mehrfach ungesättigte Heterocyclen mit 5 - 14 Ringatomen darunter 1 - 4 Heteroatome, die vorzugsweise N, O und S sind,
- C₁₋₁₂Alkylacyl (ggf. mit R^{§} substituiert),
- Benzoyl, 1- und 2-Naphthoyl (jeweils ggf. mit R^{§} substituiert),
- Heterocyclylacyl [z.B. Nicotinoyl, Isonicotinoyl, 2-Picolinoyl, 2-Thienoyl, 2-Furoyl] (ggf. mit R^{§} substituiert)
- Hydroxy,
- Sulfhydryl,
- C₁₋₁₀ Alkoxy,
- Alkylthio, Alkylsulfinyl und Alkylsulfonyl (jeweils C₁₋₆)
- Formyl, Carboxyl, C₁₋₄Alkoxycarbonyl;
- CONH₂, CONHAIk und CONAlk₂ (mit "Alk" jeweils C₁₋₆),
- Cyano, Rhodano, Nitro, SO₃H, SO₂OAlk (mit "Alk": C₁₋₅),
- Chlor, Brom, lod, Fluor,
- Amino, C₁₋₆Alkylamino, Di(C₁₋₅)alkylamino (jeweils ggf. mit R^{§} am Alkylrest substituiert),
- Morpholino, Thiomorpholino, Thiomorpholino-S,S-Dioxid, Pyrrolidino,
- Piperidino, 1-Piperazino, 4-Methyl-1-piperazino, 4-Hydroxyethyl-1-piperazino, 4-Phenyl-1-piperazino,
- Cycloalkylamino, C₃₋₁₄ Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo- bzw. heterocyclischen Ringen mit R^{§} substituiert);
- R²: - Wasserstoff,
- C₁₋₁₂ Alkyl,
- C₂₋₁₂ Alkenyl und C₂₋₁₂ Alkinyl,
- Benzyl, Phenyl(C₂₋₆)alkyl (ggf. mit R^{§} ein oder mehrmals, gleich oder ungleich am aromatischen und/oder aliphatischen Molekülteil substituiert);
- Phenacyl (ggf. mit R^{§} ein oder mehrmals, gleich oder ungleich am aromatischen Molekülteil substituiert);
- Carboxyl, C₁₋₄ Alkoxycarbonyl, CONH₂, CONHAIk und CONAlk₂ (mit "Alk" jeweils C₁-6),
- R*CO- (worin R* Wasserstoff, C₁-C₁₂Alkyl bedeuten sowie ggf. mit R^{§} substituiert),
- Cyano, Nitro, Amino, C₁₋₆Alkylamino, Di (C₁₋₆ )alkylamino, -N=N-C₆H₅, -N=N-C₆H₄-R^{§},
- 1,3-Diphenyl-pyrazol-4-yl, Thiazolin-2-yl, Imidazolin-2-yl und 3,4,5,6-Tetrahydro-pyrimidinyl;
- R⁴: - C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten aliphatischen oder cycloaliphatischen Reste mit R^{§} substituiert);
- Phenyl, 2-R^{§}-Phenyl, 3-R^{§}-Phenyl, 4-R^{§}-Phenyl, 2-R^{§},5-R^{§}-Phenyl, 3-R^{§},5-R^{§}-Phenyl, 3-R^{§},4-R^{§}-Phenyl, 3-R^{§},4-R^{§},5-R^{§}-Phenyl, 2-R^{§},3-R^{§},4-R^{§}-Phenyl,
- 1-Naphthyl, 2-Naphthyl (jeweils ggf. mit R^{§} substituiert);
- mono-, bi- oder tricyclische gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert);
- CH₂OAlk* (mit Alk*: C₁₋₆Alkyl),
- CH₂OCOR' (mit R': C₁₋₆Alkyl, C₂₋₆Alkenyl, Phenyl sowie mit R^{§} substituiert),
- COOH, COOAlk** (mit Alk**: C₁₋₅Alkyl),
- NH₂, NHAlk*, (mit Alk*: C₁₋₆Alkyl)
- NHNH₂, NHNHCOR' (mit R': C₁₋₆Alkyl, C₁₋₆Alkenyl, Phenyl sowie mit R^{§} substituiert),
- SO₃H, S-Phenacyl, S-Alkyl, SO-Alkyl, SO₂-Alkyl (jeweils C₁-C₈) S-Alkenyl, SO-Alkenyl, SO₂-Alkenyl ( jeweils C₂-C₈) S-Alkinyl, SO-Alkinyl, SO₂-Alkinyl (jeweils C₂-C₆) (ggf. jeweils am C-Skelett der oben genannten aliphatischen Reste mit -OH, -CN, -SCN, -NO₂, Phenyl oder (C₃-C₇)Cycloalkyl substituiert)

Der oben erwähnte Ausdruck "ggf. mit R^{§} substituiert" bedeutet, daß die genannten Reste einfach oder mehrfach, gleich oder ungleich unabhängig voneinander, substituiert sein können, wobei R^{§} folgende Bedeutung hat:
-OH, -SH, -O-C₁₋₈Alkyl, -O-C₆₋₁₄ Aryl, -S-C₁₋₄Alkyl, -S-C₆₋₁₄Aryl, -SO-C₁₋₄Alkyl, -SO-C₆₋₁₄Aryl, -SO₂-C₁₋₄Alkyl, -SO₂-C₆₋₁₄Aryl, -SO₃H, -OSO₂C₁₋₈Alkyl, -OSO₂C₆₋₁₄Aryl, -COOH, -COOC₁₋₈Alkyl, -(CO)C₁₋₈Alkyl, -COOH, -COOC_{1...8}Alkyl, -CONH₂, -CONHC₁₋₆Alkyl, -CON(C₁₋₆Alkyl)₂, -NH₂, -NHC₁₋₆Alkyl, -N(C₁₋₆Alkyl)₂, -NHC₆₋₁₄Aryl, -NH-Hetaryl, -N(C₆₋₁₄Aryl)₂, -N(C₁₋₆Alkyl)(C₆₋₁₄Aryl),
-CH₃, -CHF₂, -CF₃, -C₂H₅, -C(CH₃)₂, -(CH₂)₂CH₃, -(CH₂)₃CH₃, -CH₂CH₂OH, -CH₂CH₂SH, -CH₂CH₂SCH₃, -CH₂CF₃, -CH₂CC1₃, -CH₂CHF₂, -CH₂CHCl₂, -CH₂CH₂F, -CH₂CH₂Cl, -CH₂CH₂Br, -Cylopropyl, -Cylopropylmethyl, -Cylobutyl, -Cyclobutylmethyl, -Cyclopentyl, -Cyclopentylmethyl, -Cylohexyl, -Cyclohexylmethyl,
-F, -Cl, -Br, -1, -CN, -NO₂, und -SCN.

Ausgenommen vom Schutz sind die folgenden Verbindungen der Formel 1a:

| | R¹ | R² | R³ |
|---|---|---|---|
| 1. | Me | H | Me |
| 2. | Me | Ac | 4-NMe₂Ph |
| 3. | Me | Ac | 4-NO₂Ph |

Ausgenommen vom Schutz sind auch die folgenden Verbindungen der Formel 1b mit folgenden Resten:
jeweils R¹ = OEt, R² = CN, R³ = Ph mit R⁴ = Benzyl, Phenyl, z.T. mehrfach mit Me, OMe, NO₂ und Cl substituiert und 3,4-Methylendioxy-phenyl;
jeweils R¹ = Ph, R² = H, R³ = Ph mit R⁴ = Phenyl, p-OCH₃-Ph, p-Cl-Ph, 2,3,4-Tri-OCH₃-Ph und 2-Thienyl;
jeweils R¹ = Me, R² = H, R³ = Me mit R⁴ = CH₂OMe, CH₂OEt, CH₂OCOMe, CH₂COOPh, SCH₂COPh, NH₂, NHNH₂, COOH, Ph; sowie:
   R¹ = Me, R² = H, R³ = Ph, R⁴ = COOEt;
   R¹ = Me, R² = H, R³ = p-Br-Ph, R⁴ = COOEt;
   R¹ = p-Br-Ph, R² = H, R³ = Me, R⁴ = COOMe;
   R¹ = Me, R² = Benzyl, R³ = Me, R⁴ = COOMe;
   R¹ = R² = R³ = Me, R⁴ = COOMe.

Die Begriffe "Alkyl, Alkenyl, Alkinyl, Alkoxy, usw.", auch in Wortzusammensetzungen wie Alkylsulfonyl, Alkylamino oder Alkoxycarbonyl usw. bedeuten sowohl die unverzweigten wie auch die verzweigten möglichen Verbindungen. Ebenso bedeuten "Alkenyl und Alkinyl" die entsprechend möglichen einfach oder mehrfach ungesättigten Verbindungen. Das gleiche gilt auch für die entsprechenden cyclischen Verbindungen.

Die Erfindung betrifft auch physiologisch verträgliche Salze der Verbindungen der allgemeinen Formeln 1a und 1b.

Die physiologisch verträglichen Salze werden auf übliche Weise durch Umsetzung basischer Verbindungen der allgemeinen Formeln 1a und 1b mit anorganischen oder organischen Säuren, ggf. auch bei Vorliegen von Verbindungen mit aciden Eigenschaften, wenn z.B. einer der Substituenten R¹, R², R³ oder R⁴ in diesen Verbindungen -COOH bzw. -SO₃H bedeutet, durch Neutralisation mit anorganischen oder organischen Basen, erhalten.

Als anorganische Säuren kommen vorzugsweise Salzsäure, Schwefelsäure, Salpetersäure oder Bromwasserstoffsäure, als organische Säuren zum Beispiel Ameisensäure, Essigsäure, Propionsäure, Glykolsäure, Milchsäure, Mandelsäure, Weinsäure, Äpfelsäure, Zitronensäure, Malonsäure, Maleinsäure, Fumarsäure, Succinsäure, Alginsäure, Benzoesäure, 2-, 3- und 4-Alkyloxy- und Acyloxybenzoesäuren, Ascorbinsäure, C₁-C₃,Alkylsulfonsäuren, Benzolsulfonsäure, Nicotinsäure, Isonicotinsäure und Aminosäuren zur Anwendung.

Als anorganische Basen kommen zum Beispiel Ammoniak, Natron- und Kalilauge sowie als organische Basen Alkylamine, C₁-C₃, Pyridin, Chinolin, Isochinolin, Piperazin und -Derivate, Picoline, Chinaldin oder Pyrimidin zur Anwendung.

Weiterhin können physiologisch verträgliche Salze der Verbindungen gemäß der allgemeinen Formeln 1a und 1b dadurch gewonnen werden, dass jene Substanzen, die als Substituenten eine tertiäre Amino-Gruppe besitzen, in prinzipiell bekannter Weise mit alkylierenden Agentien - wie zum Beispiel Alkyl- oder Aralkylhalogeniden - in die entsprechenden quarternären Ammoniumsalze übergeführt werden können.

Die Erfindung betrifft auch Solvate der Verbindungen, einschließlich der pharmazeutisch akzeptablen Salze, Säuren, Basen und Ester sowie deren aktive Metabolite und gegebenenfalls deren Tautomere gemäß der allgemeinen Formeln 1a und 1b einschließlich Prodrug-Formulierungen. Prodrug-Formulierungen umfassen hierbei alle jene Substanzen, die durch einfache Transformation einschließlich Hydrolyse, Oxidation, oder Reduktion entweder enzymatisch, metabolisch oder auf andere Art und Weise entstehen. Insbesondere, wenn ein solches Prodrug dieser erfindungsgemäßen Verbindungen einem Patienten appliziert wurde, und dieses Prodrug in eine Substanz der allgemeinen Formeln 1a, und 1b transformiert wird, wodurch der gewünschte pharmakologische Effekt erzielt wird.

Die durch die erfindungsgemäßen Verbindungen behandelbaren Erkrankungen schließen alle ein, bei denen TNF-alpha eine Rolle spielt und die durch eine Hemmung oder Inhibierung desselben positiv zu beeinflussen sind, z.B. chronische Entzündungserkrankungen, Autoimmun-Erkrankungen, cardiovaskuläre Erkrankungen, virale Erkrankungen und hier insbesondere retrovirale Erkrankungen wie z.B. das erworbene lmmundefizienz Syndrom (AIDS) sowie Krebs, insbesondere Entartungen des blutbildenden Systems. Insbesondere sind dies Rheumatoide Arthritis, Osteoarthritis, Osteoporosis, Asthma bronchiale, chronische obstruktive pulmonäre Erkrankung (COPD), Multiple Sklerose, Sepsis, cerebrale Form der Malaria, neurodegenerativen Erkrankungen wie z.B. Mb. Alzheimer, Mb. Parkinson, Guillain-Barre-Syndrom, Crohns Disease, Colitis ulcerosa, Psoriasis, Graft-versus-Host-Disease (GvHD), systemischer Lupus erythomatodes (SLE), Vasculitis, Uveitis, insulin-abhängiger Diabetes mellitus, Respiratorisches Distress-Syndrom beim Erwachsenen (ARDS), multiples Organversagen nach Trauma, aktute Glomerulonephritis, akute und chronische Schmerzen, Arteriosklerose, Herzinfarkt, Schlaganfall, entzündliche Dermatosen, atopische Dermatitis, Psoriasis vulgaris, Alopecie, Rhinitis allergica, allergische Konjunktivitis, akute Meningitis, Myastenia Gravis, Sklerodermie und Sarkoidose.

Die erfindungsgemäßen Verbindungen können auf verschieden Wegen verabreicht werden, z.B. oral, parenteral, kutan, subkutan, intravenös, intramuskulär, rektal oder inhalativ. Bevorzugt ist die intravenöse oder inhalative Verabreichung. Die Verbindung wird einem Patienten, der eine Therapie einer unter das Indikationsspektrum der erfindungsgemäßen Verbindungen fallenden Krankheit bedarf, über einen vom Arzt zu bestimmenden Zeitraum verabreicht. Die Verbindung kann sowohl Menschen als auch anderen Säugern verabreicht werden.

Die Dosierung der erfindungsgemäßen Verbindungen wird vom Arzt anhand der patientenspezifischen Parameter wie z.B. Alter, Gewicht, Geschlecht, Schwere der Erkrankung, etc. bestimmt. Bevorzugt beträgt die Dosierung zwischen 0,001 mg/kg bis 100.000 mg/kg Körpergewicht, bevorzugt 0,01 bis 10.000 mg/kg Körpergewicht und ganz bevorzugt 0,1 bis 1.000 mg/kg Körpergewicht.

Entsprechend der Art der Verabreichung wird das Medikament in geeigneter Weise formuliert, z.B. in Form von Lösungen bzw. Suspensionen, einfachen oder dragierten Tabletten, Hart- oder Weichgelatinekapseln, Pulver zur Rekonstitution vor Gebrauch, Aerosolen, Inhalationssprays, Wirkstoffpflastern, Granulaten, Suppositorien, Ovula, Injektionspräparaten, Cremes, Salben, Gels, Mikrospheren, Implantaten, die nach üblichen galenischen Verfahren hergestellt werden.

Die erfindungsgemäßen Verbindungen können gegebenenfalls zusammen mit weiteren Wirkstoffen und mit in pharmazeutischen Zusammensetzungen üblichen Exzipientien formuliert werden, z.B. je nach herzustellendem Präparat Talk, Gummi arabicum, Lactose, Stärke, Magnesiumstearat, Kakaobutter, wäßrige und nichtwäßrige Träger, Fettkörper mit tierischem oder pflanzlichem Ursprung, Paraffinderivate, Glykole (insbesondere Polytethylenglykol), verschiedene Weichmacher, Dispergiermittel oder Emulgatoren, pharmazeutisch verträgliche Gase (z.B. Luft, Sauerstoff, Kohlendioxid usw.), Konservierungsstoffe.

Zur Herstellung flüssiger Präparate können Additive wie Natriumchloridlösung, Ethanol, Sorbit, Glycerin, Olivenöl, Mandelöl, Propylenglycol oder Ethylenglycol verwendet werden.

Bei der Verwendung von Infusions- oder Injektionslösungen sind diese sind bevorzugt wäßrige Lösungen oder Suspensionen, wobei es möglich ist, diese vor Gebrauch herzustellen, beispielsweise aus lyophilisierten Präparaten, die den Wirkstoff alleine oder zusammen mit einem Träger, wie Mannit, Lactose, Glucose, Albumin und dergleichen, enthalten. Die gebrauchsfertigen Lösungen werden sterilisiert und gegebenenfalls mit Hilfsmitteln vermischt, beispielsweise mit Konservierungsstoffen, Stabilisatoren, Emulgatoren, Lösungsvermittlern, Puffern und/oder Salzen zur Regulierung des osmotischen Drucks. Die Sterilisierung kann durch Sterilfiltration durch Filter mit einer kleinen Porengröße erzielt werden, wonach die Zusammensetzung gegebenenfalls lyophilisiert werden kann. Geringe Mengen an Antibiotika können auch zugesetzt werden, um die Beibehaltung der Sterilität zu gewährleisten.

Weiter bevorzugt werden Inhalationszusammensetzungen, z.B. in Form von Aerosolen, Sprays, oder als mikronisiertes Pulver hergestellt. Dazu werden die erfindungsgemäßen Verbindungen entweder als in pharmazeutisch üblichen Lösungsmitteln gelöst bzw. suspendiert und mittels Überdruck in einem bestimmten Volumen fein verteilt und inhaliert. Ein entsprechendes Vorgehen erfolgt bei den zu inhalierenden Festsubstanzen, die gleichfalls mittels Überdruck fein verteilt und inhaliert werden. Ebenfalls andere als mit Überdruck funktionierende Applikatoren sind hierbei eingeschlossen.

Die Erfindung betrifft auch pharmazeutische Zubereitungen, die eine therapeutisch wirksame Menge des aktiven Inhaltsstoffs (erfindungsgemäße Verbindung der Formel (1a) oder (1b)) zusammen mit organischen oder anorganischen festen oder flüssigen pharmazeutisch verträglichen Trägern, die für die beabsichtigte Verabreichung geeignet sind, und die mit den aktiven Inhaltsstoffen nicht nachteilig wechselwirken, enthalten.

Die Erfindung betrifft auch Verfahren zur Herstellung pharmazeutischer Zubereitungen, die dadurch gekennzeichnet sind, dass die erfindungsgemäße Verbindung mit einem pharmazeutisch verträglichen Träger vermischt wird.

Die erfindungsgemäßen Verbindungen eignen sich auch im Rahmen von Kombinationstherapien mit schon bekannten Wirkstoffen zur Behandlung der oben genannten Erkrankungen. Dabei sollen überraschende Synergieeffekte zur Steigerung der therapeutischen Wirksamkeit der erfindungsgemäßen Substanzen genutzt werden. Die Kombination kann zum einen darin bestehen, eine einzige pharmazeutische Zusammensetzung anzubieten, die mindestens eine der erfindungsgemäßen Verbindungen in Kombination mit einem oder mehrere der nachfolgend genannten Wirkstoffen enthält oder dem Patienten werden gleichzeitig oder zeitlich versetzt mehrere Mittel, die einen oder mehreren der nachfolgenden Wirkstoffe enthalten, verabreicht.

Es ist bevorzugt eine oder mehrere der erfindungsgemäßen Verbindungen mit einem oder mehreren der folgenden Wirkstoffe zu kombinieren:
- Corticosteroide
- (monoklonale) Antikörpern gegen TNF-alpha oder andere Wirkstoffe, die die Bildung bzw. Freisetzung von TNF-alpha oder die Aktivität von TNF-alpha hemmen (z.B. rekombinante TNFα-Rezeptorkonstrukte)
- Zytokin-Antagonisten (z.B. IL-1β, IL-6, IL-12)
- Chemokin-Antagonisten
- Zytokin-Agonisten (z.B. IL-10)
- immunmodulatorische Wirkstoffe wie z.B. Cyclosporin A, Methodrexat, Leflunomid, D-Penicillamin, Auranofine
- Substanz P-Antagonisten
- Bradykinin-Antagonisten
- PAF-Antagonisten
- Adenosin-Rezeptor-Antagonisten
- Antibiotika/Virostatika
- α-Mimetika
- Zytostatika
- β₂-Adrenoceptor Agonisten (z.B. Terbutalin, Salbutanol, Salmetanol, Fenoterol, Formoterol)
- Leukotrien-Antagonisten (entweder Enzym-Inhibitoren [wie 5-Lipoxygenaseinhibitoren oder Arachidonsäure-Enzyminhibitoren] oder Rezeptorantagonisten), z.B. Pranlukast, Montelukast, Zafirlukast, Zileuton
- Antihistaminika (bevorzugt solche mit Mastzellen-stabilisierenden Eigenschaften oder Leukotrien-antagonisierenden Aspekten, wie z.B. Loratadin, Astemizol, Mizolastin, , Olopatadin
- Theophyllin
- Muscarinrezeptor-Antagonisten, z.B. Spiriva

Die Kombination mit oben aufgeführten Arzneimitteln bzw. Wirkprinzipien dient besonders dazu, den akut zu behandelnden Krankheitszustand in einem möglichst frühen Stadium in seiner Manifestation zu beeinflussen und nicht chronisch werden zu lassen, da die erfindungsgemäßen Verbindungen in Kombination mit den anderen Wirkstoffen komplementäre/additive Aspekte ermöglichen. In der Kombination ergibt sich ein positiver Effekt u.a. daraus, dass eine geringere Substanzmenge pro Prinzip angewendet werden kann und damit zum einen eine Verbesserung des therapeutischen Effektes, geringere UAWs und zum anderen ein Spareffekt zu erreichen ist.

Abhängig von der Krankheitsausprägung und den zugrunde liegenden Symptomen können die erfindungsgemäßen Verbindungen zu den anderen Wirkstoffen in der Kombination im Verhältnis von 1:10.000 bis 10.000:1 vorliegen.

Die Erfindung betrifft weiterhin Verfahren zur Herstellung der erfindungsgemäßen Verbindungen.

Die erfindungsgemäßen Verfahren zur Herstellung der Verbindungen der allgemeinen Formeln 1a und 1b mit den zuvor aufgeführten Bedeutungen von R¹, R², R³, R⁴, und Y sind gekennzeichnet durch folgende Verfahrensweisen:
A) - Umsetzung der bekannten bzw. nach prinzipiell bekannten Methoden dargestellten Pyridin-3-carbonitrile der allgemeinen Formel 2, 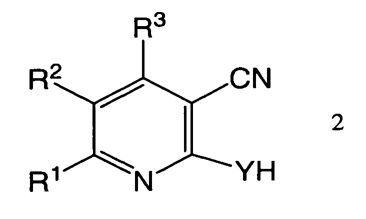
   mit identischer Bedeutung von R¹, R², R³ und Y wie oben,
   in an sich bekannter Weise mit Chloracetamid, CICH₂CONH₂, in methanolischer oder ethanolischer Lösung in Gegenwart eines Natriumalkoxides, vorzugsweise Natriummethoxid oder Natriumethoxid, zunächst zu den analogen 3-Aminothieno[2,3-b]pyridin-2-carbonsäureamiden der allgemeinen Formel 4,
   mit identischer Bedeutung von R¹, R², R³ und worin Y die Bedeutung von S aufweist, bzw. in die analogen 3-Amino-furo[2,3-b]pyridin-2-carbonsäureamide der allgemeinen Formel 4, 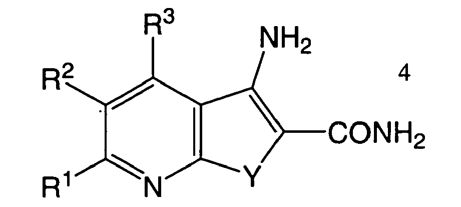
   mit identischer Bedeutung von R¹, R² und R³ und worin Y die Bedeutung von O aufweist;
   - Die Verbindungen der allgemeinen Formel 4 (mit identischer Bedeutung von R¹, R², R³ und Y wie oben) sind aus den Verbindungen der allgemeinen Formel 2, auch dadurch darstellbar, dass diese Verbindungen mit Choracetamid zunächst in vorzugsweise ethanolischer Lösung in Gegenwart von vorzugsweise Triethylamin oder in wasserfreier acetonischer Lösung in Gegenwart von Natrium- oder Kaliumhydrogencarbonat zu den Verbindungen der allgemeinen Formel 3, 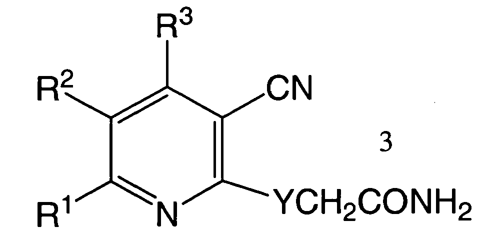
      worin R¹, R², R³ und Y die oben genannten Bedeutungen aufweisen, umgesetzt werden
      und diese Verbindungen in einem weiteren Syntheseschritt in vorzugsweise wasserfreier ethanolischer Lösung mit einer katalytischen Menge Natriummethoxid oder Natriumethoxid durch Erhitzen unter Rückfluss gleichfalls in die oben genannten Verbindungen der allgemeinen Formel 4 übergeführt werden;
   - Umsetzung der Verbindungen der allgemeinen Formel 4 mit Phosgen, Trichlormethylchlorformiat oder mit Alkylchlorformiaten der allgemeinen Formel 5,

      ClCOOR^{§§} 5,

      worin R^{§§} C₁₋₃-Alkyl bedeutet,
      in einem vorzugsweise aprotischen, hochsiedenden Lösungsmittel wie Dioxan, Tetrahydrofuran oder Toluol bzw. deren Mischungen unter Erhitzen, ggf. in Anwesenheit katalytischer Mengen eines Natriumalkoxides, wie zum Beispiel Natriumethylat, zu Verbindungen der allgemeinen Formel 1a, 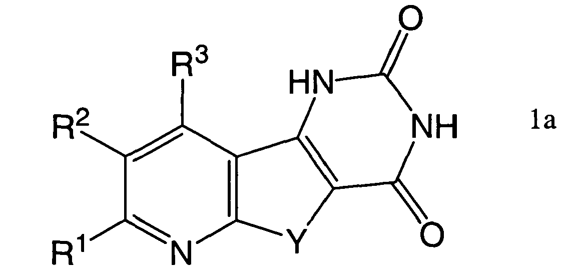
      worin R¹, R², R³ und Y dasselbe wie oben bedeuten;
      oder
      B)
   - Umsetzung der Verbindungen der allgemeinen Formel 4,
      worin R¹, R², R³ und Y dasselbe wie oben bedeuten,
      mit Carbonsäurehalogeniden der allgemeinen Formel 6,

      R⁴-CO-Z 6

worin
- R⁴: C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten aliphatischen oder cycloaliphatischen Reste mit R^{§} substituiert);
- Phenyl, 2-R^{§}-Phenyl, 3-R^{§}-Phenyl, 4-R^{§}-Phenyl, 2-R^{§},5-R^{§}-Phenyl, 3-R^{§},5-R^{§}-Phenyl, 3-R^{§},4-R^{§}-Phenyl, 3-R^{§},4-R^{§},5-R^{§}-Phenyl, 2-R^{§},3-R^{§},4-R^{§}-Phenyl,
- 1-Naphthyl, 2-Naphthyl (jeweils ggf. mit R^{§} substituiert);
- mono-, bi- oder tricyclische gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert);
- CH₂OAlk* (mit Alk*: C₁₋₆Alkyl),
- CH₂OCOR' (mit R': C₁₋₆Alkyl, C₂₋₆Alkenyl, Phenyl sowie mit R^{§} substituiert),
- COOH, COOAlk** (mit Alk**: C₁₋₅Alkyl),

bedeutet
und
Z = Chlor oder Brom

oder
- Umsetzung der Verbindungen der allgemeinen Formel 4 (worin R¹, R², R³ und Y dasselbe wie oben bedeuten),
   mit Carbonsäureanhydriden der allgemeinen Formel 7,

   (R⁴CO)₂O 7

   worin R⁴ dasselbe wie bei den Verbindungen der allgemeinen Formel 6 bedeutet, jeweils im Überschuss und in der Siedehitze,
   gegebenenfalls zusammen mit Pyridin oder einem aprotischen, hochsiedenden Lösungsmittel, wie zum Beispiel Toluol oder Xylol,
   Behandlung des gebildeten Niederschlages mit wässerig-ethanolischer Natronlauge oder mittels konz. wässeriger Ammoniak-Lösung, jeweils bis zu einem pH-Wert von 8-10, unter Erwärmen,
   nachfolgendem Ansäuern mit verdünnter Salzsäure unter Bildung der Verbindungen der allgemeinen Formel 1b, 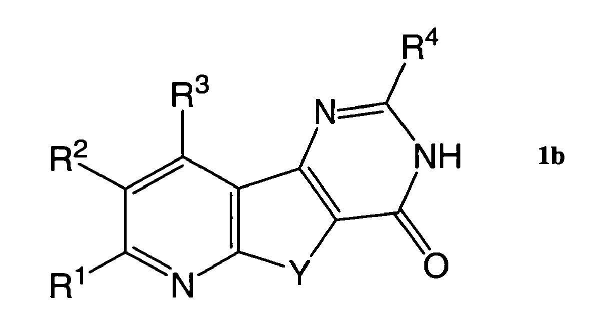
   worin R¹, R², R³ und Y dasselbe wie oben bedeuten und R⁴ die bei den Verbindungen der allgemeinen Formel 6 angegebene Bedeutung aufweist
   oder
   C)
- Umsetzung der aus den Verbindungen der allgemeinen Formel 2 und Chloressigsäurealkylestern, C₁-C₃, darstellbaren bekannten
   oder nach prinzipiell bekannten Methoden darstellbaren Verbindungen der allgemeinen Formel 8, 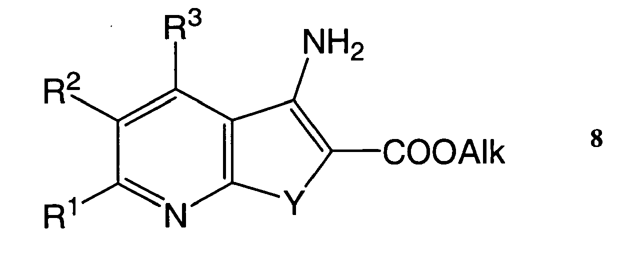
   worin R¹, R², R³, Y dasselbe wie oben und Alk Alkyl, C₁-C₃, bedeuten,
   mit Benzoylisothiocyanat, PhCONCS, in aprotischen, dipolaren Lösungsmitteln, vorzugsweise Aceton oder Dioxan
   unter Erwärmen zu den Verbindungen der allgemeinen Formel 9, 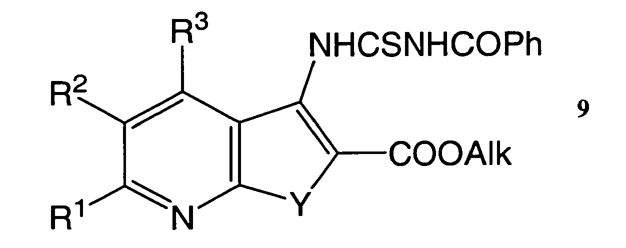
   worin R¹, R², R³, Y dasselbe wie oben und Alk Alkyl, C₁-C₃, bedeuten,
- Umsetzung der Verbindungen der allgemeinen Formel 9 mit Natrium- oder Kaliumhydroxid in protischen oder aprotischen polaren Lösungsmitteln oder Lösungsmittelgemischen unter Erwärmen,
   Zugabe von Brom- oder lodalkanen, -alkenen oder alkinen, C₃-C₇zu der erkalteten alkalischen Reaktionslösung,
   mäßiges Erwärmen bis zur Beendigung der Umsetzung,
   Ansäuern mit verdünnter Salzsäure unter Entstehung der Verbindungen der allgemeinen Formel 1b, 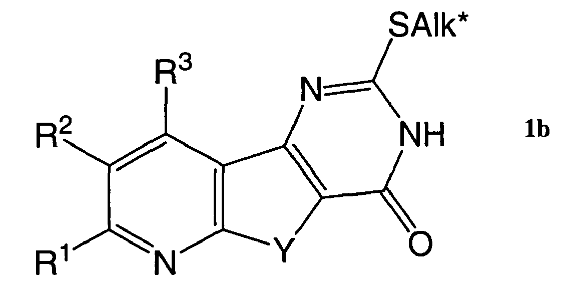
   worin R¹, R², R³, Y dasselbe wie oben und Alk* (C₁₋₈)Alkyl, (C₂₋₈,)Alkenyl, (C₃-₆) Alkinyl, jeweils unverzweigt oder gegebenenfalls verzweigt und ggf. mit einem Rest wie -CN, -SCN, NO₂, Phenyl und (C₃₋₇)Cycloalkyl substituiert ist, bedeuten,
   oder
   D)
- Umsetzung von Verbindungen der allgemeinen Formel 1b bei denen R⁴ SAlk* und Alk* (C₁₋₈)Alkyl**,** (C₂₋₈,)Alkenyl, (C₃₋₆) Alkinyl, jeweils unverzweigt oder verzweigt und ggf. mit einem Rest wie -CN, -SCN, NO₂, Phenyl und (C₃₋₇)Cycloalkyl substituiert ist, bedeutet,
   mit annähernd äquimolarer Menge Dihydrogenperoxid in Essigsäure oder Ameisensäure unter dc-Kontrolle bei Raumtemperatur
   oder mit KIO₄ in protischen oder aprotischen, dipolaren Lösungsmitteln unter Erwärmen
   zu jenen Verbindungen der allgemeinen Formel 1b,
   worin R¹, R², R³ und Y dasselbe wie oben bedeuten
   und R⁴ SOAlk* und Alk* (C₁₋₈)Alkyl, (C₂₋₈,)Alkenyl, (C₃₋₆) Alkinyl, jeweils unverzweigt oder verzweigt und ggf. mit einem Rest wie -CN, -SCN, NO₂, Phenyl und (C₃₋₇)Cycloalkyl substituiert ist, bedeutet
   oder
   E)
- Umsetzung jener Verbindungen der allgemeinen Formel 1b bei denen R⁴ SAlk* oder SOAlk* und Alk* (C₁₋₈)Alkyl, (C₂₋₈,)Alkenyl, (C₃₋₆) Alkinyl, jeweils unverzweigt oder verzweigt und ggf. mit einem Rest wie -CN, -SCN, NO₂, Phenyl und (C₃₋₇)Cycloalkyl substituiert ist, bedeutet,
   mit überschüssigem Dihydrogenperoxid in Essigsäure oder Ameisensäure unter dc-Kontrolle bei Raumtemperatur, gegebenenfalls auch unter mäßigem Erwärmen oder mit KMnO₄ in protischen oder aprotischen, dipolaren Lösungsmitteln unter Erwärmen
   zu den Verbindungen der allgemeinen Formel 1b,

worin R¹, R², R³ und Y dasselbe wie oben bedeuten und R⁴SO₂AIk* und Alk*(C₁₋₈) Alkyl, (C₂₋₈,)Alkenyl, (C₃₋₆) Alkinyl, jeweils unverzweigt oder verzweigt und ggf. mit einem Rest wie -CN, -SCN, NO₂, Phenyl und (C₃₋₇)Cycloalkyl substituiert ist, bedeutet
Entsprechend der vorliegenden Erfindung können die unter A) bis E) beschriebenen Verfahren innerhalb weiter Grenzen variiert werden.

Weitere Ausführungsformen der Erfindung können beispielsweise darin bestehen, dass die Darstellung der tricyclischen Pyrimidin-2,4-dione der allgemeinen Formel 1a bzw. die der tricyclischen Pyrimidin-4-one der allgemeinen Formel 1a unter Verwendung einer Mikrowelle realisiert wird,

### Methodenbeschreibung der Hemmung der TNFα Freisetzung nach LPS Stimulation von humanem Vollblut

Die Stimulierung isolierter Leukozyten für die Freisetzung von Zytokinen kann auf verschiedenen Wegen erfolgen. Lipopolysaccharide (LPS) stellen einen Stimulus für die Untersuchung der Freisetzung von TNFα dar. LPS ist Bestandteil bakterieller Zellwände und wird beim Abtöten der Bakterien (durch Antibiotika oder das natürliche Immunsystem) freigesetzt. LPS stimuliert insbesondere die Aktivität phagozytierender Leukozyten (Gewebsmakrophagen, Granulozyten, Monozyten) und verursacht die Infiltration von Leukozyten vom peripheren Blut in das betroffene Gewebe. Ein Zytokin von besonderer Bedeutung für diese Mechanismen ist TNFα, das in großen Mengen durch die betroffenen Zellen sezerniert wird. Hauptquelle dabei sind Monozyten und Makrophagen. TNFα initiiert und prolongiert den Entzündungsprozess im Zusammenspiel mit anderen Mediatoren.

Für die Untersuchung des Effektes auf die LPS-induzierte TNFα-Freisetzung wurde eine Methode verwendet, die von MARX et al. (*Marx D, Tassabehji M, Heer S, Hüttenbrink KB, Szelenyi I (2002) Pulmonary Pharmacology & Therapeutics 15 7-15)* beschrieben wurde. Die Methode in Kürze: Humanes Blut wurde von verschiedenen Spendern entnommen, durch Zusatz von 10 mM Na-Citrat ungerinnbar gemacht und 1:5 mit RPMI 1640 Zellkulturmedium verdünnt. Die Testsubstanzen wurden den Blutproben in verschiedenen Konzentrationen zugefügt. 15 Minuten später wurden die Leukozyten durch Zusatz von Lipopolysacchariden (LPS) aus Salmonella abortus equi in einer Endkonzentration von 1µg/ml stimuliert. Nach Inkubation der Testansätze für 24 Stunden bei 37°C. und unter 5% CO₂ in wassergesättigter Luft, wurde das Blut zentrifugiert und die Konzentration an TNFα im zellfreien Überstand unter Verwendung eines käuflichen ELISA (BD Biosciences) nach Angaben des Herstellers exakt vermessen.

EC₅₀-Werte im Bereich von 10⁻⁹ bis 10⁻¹⁰ M wurden für die in der Erfindung beschriebenen Substanzen bestimmt.

Die Verbindungen der allgemeinen Formeln 1a und 1b sind schwache Inhibitoren der Phosphodiesterase 4 und äußerst starke Inhibitoren der Freisetzung von TNFα.

Die nachfolgende Auflistung beinhaltet erfindungsgemäße Substanzen der allgemeinen Formeln 1a und 1b, die bei 100nM die TNFα-Freisetzung >10% hemmen :
9-Methyl-2-methylthio-7-phenyl-pyrido[3',2':4,5]thieno[3,2-d]pyrimidin-4(3H)-on;
9-Methyl-8-(4-nitrophenyl)-7-phenyl-pyrido[3',2':4,5]thieno[3,2-d]pyrimidin-2,4(1H,3H)-dion;
7-Phenyl-pyrido[3',2':4,5]thieno[3,2-d]pyrimidin-4(3H)-on-2-carbonsäureethylester;
7,9-Diphenyl-pyrido[3',2':4,5]thieno[3,2-d]pyrimidin-2,4(1H,3H)-dion;
7-(4-Brom-phenyl)-2,9-dimethyl pyrido[3',2':4,5]thieno[3,2-d]pyrimidin-4(3H)-on

Die nachfolgende Auflistung beinhaltet erfindungsgemäße Substanzen der allgemeinen Formeln 1a und 1b, die bei 10nM die TNFα-Freisetzung >20% hemmen :
2-Ethyl-9-methyl-7-phenyl-pyrido[3',2':4,5]thieno[3,2-d]pyrimidin-4(3H)-on;
7-(3,4-Dimethoxy)-9-methyl-pyrido[3',2':4,5]thieno[3,2-d]pyrimidin-2,4(1H,3H)-dion;
7-Phenyl-2-n-propyl-9-(pyrid-3-yl)-pyrido[3',2':4,5]thieno[3,2-d]pyrimidin-4(3H)-on;
7-(4-Fluor-phenyl)-9-methyl-pyrido[3',2':4,5]thieno[3,2-d]pyrimidin-2,4(1H,3H)-dion;
7-(4-Methoxy-phenyl)-9-methyl-pyrido[3',2':4,5]thieno[3,2-d]pyrimidin-2,4(1H,3H)-dion;
7-Phenyl-pyrido[3',2':4,5]thieno[3,2-d]pyrimidin-4(3H)-on-2-carbonsäure;
9-Methyl-7-phenyl-pyrido[3',2':4,5]thieno[3,2-d]pyrimidin-2,4(1H,3H)-dion;
7-Phenyl-pyrido[3',2':4,5]thieno[3,2-d]pyrimidin-4(3H)-on-2-carbonsäure;
7,9-Dimethyl-pyrido[3',2':4,5]thieno[3,2-d]pyrimidin-2,4(1H,3H)-dion;

Die Erfindung wird durch die nachfolgenden Beispiele näher erläutert:

### Beispiel 1

### 7-(3,4-Dimethoxyphenyl)-9-methyl-pyrido[3',2':4,5]thieno[3,2-d]pyrimidin-2,4-dion (allgemeine Formel 1a: R₁=3,4-(MeO)₂-Ph, R₂=H, R₃=Me, Y=S)

3.0 g (8.7 mmol) 3-Amino-6-(3,4-dimethoxyphenyl)-4-methyl-thieno[2,3-b]pyridin-2-carbonsäureamid (Beispiel 4a; allgemeine Formel 4: R₁=3,4-(CH₃O)₂-Ph, R₂=H, R₃=Me, Y=S) werden in 100 ml abs. Dioxan suspendiert. Anschließend werden 1.1 ml (8.7 mmol) Chlorameisensäure-trichlormethylester (Diphosgen) zugegeben und 3 Stunden unter Rückfluß erhitzt. Nach Abkühlen wird der Niederschlag abgesaugt und anschließend in 50 ml Wasser suspendiert. Die Suspension wird mit konz. Ammoniak-Lösung auf pH 8 eingestellt und der Niederschlag abgesaugt, mit wenig Wasser gewaschen, trocken gesaugt und i. Vak. bei 50 °C getrocknet.
Ausbeute: 3.08 g (96 %); HPLC t_{R}: 13.8 min, Reinheit: 99.9 % (s. a in Tab. 1);
MS (ESI, m/e): 368 [M-H]-

Ausgehend von den nachfolgend beschriebenen Vorstufen werden durch Umsetzung der entsprechenden Verbindungen der allgemeinen Formel 4 mit Chlorameisensäuretrichlormethylester analog Beispiel 1 folgende Verbindungen (2-8) erhalten:

### Beispiel 2

### 7-(4-Methoxyphenyl)-9-methyl-pyrido[3',2':4,5]thieno[3,2-d]pyrimidin-2,4-dion (allgemeine Formel 1a: R₁=4-MeO-Ph, R₂=H, R₃=Me, Y=S)

Analog Beispiel 1 aus 3-Amino-6-(4-methoxyphenyl)-4-methyl-thieno[2,3-*b*]pyridin-2-carbonsäureamid (Beispiel 4b; allgemeine Formel 4: R₁=4-MeO-Ph, R₂=H, R₃=Me, Y=S) (s. Tab. 1)

### Beispiel 3

### 7-(4-Fluorphenyl)-9-methyl-pyrido[3',2':4,5]thieno[3,2-d]pyrimidin-2,4-dion (allgemeine Formel 1a: R₁=4-F-Ph, R₂=H, R₃=Me, Y=S)

Analog Beispiel 1 aus 3-Amino-6-(4-fluorphenyl)-4-methyl-thieno[2,3-b]pyridin-2-carbonsäureamid (Beispiel 4c; allgemeine Formel 4: R₁=4-F-Ph, R₂=H, R₃=Me, Y=S) (s. Tab. 1)

### Beispiel 4

### 9-Methyl-7-phenyl-pyrido[3',2':4,5]thieno[3,2-d]pyrimidin-2,4-dion (allgemeine Formel 1a: R₁=Ph, R₂=H, R₃=Me, Y=S)

Analog Beispiel 1 aus 3-Amino-4-methyl-6-phenyl-thieno[2,3-b]pyridin-2-carbonsäureamid (Beispiel 4d; allgemeine Formel 4: R₁=Ph, R₂=H, R₃=Me, Y=S) (s. Tab. 1)

### Beispiel 5

### 7-Methyl-9-phenyl-pyrido[3',2':4,5]thieno[3,2-d]pyrimidin-2,4-dion (Beispiel 5; allgemeine Formel 1a: R₁=Me, R₂=H, R₃=Ph, Y=S)

Analog Beispiel 1 aus 3-Amino-6-methyl-4-phenyl-thieno[2,3-b]pyridin-2-carbonsäureamid (allgemeine Formel 4: R₁=Me, R₂=H, R₃=Ph, Y=S) (s. Tab. 1)

### Beispiel 6

### 7,9-Diphenyl-pyrido[3',2':4,5]thieno[3,2-d]pyrimidin-2,4-dion (allgemeine Formel 1a: R₁=Ph, R₂=H, R₃=Ph, Y=S)

Analog Beispiel 1 aus 3-Amino-4,6-diphenyl-thieno[2,3-*b*]pyridin-2-carbonsäureamid (Beispiel 4f; allgemeine Formel 4: R₁=Ph, R₂=H, R₃=Ph, Y=S) (s. Tab. 1)

### Beispiel 7

### 7,9-Dimethyl-pyrido[3',2':4,5]thieno[3,2-d]pyrimidin-2,4-dion (allgemeine Formel 1a: R₁=Me, R₂=H, R₃=Me, Y=S)

Analog Beispiel 1 aus 3-Amino-4,6-dimethyl-thieno[2,3-*b*]pyridin-2-carbonsäureamid (Beispiel 4g; allgemeine Formel 4: R₁=Me, R₂=H, R₃=Me, Y=S) (s. Tab. 1)

### Beispiel 8

### 7,9-Dimethyl-8-(4'-nitrobenzyl)-pyrido[3',2':4,5]thieno[3,2-d]pyrimidin-2,4-dion (allgemeine Formel 1a: R₁=Me, R₂=4'-NO₂-Ph-CH₂, R₃=Me, Y=S)

Analog Beispiel 1 aus 3-Amino-4,6-dimethyl-5-(4'-nitrobenzyl)-thieno[2,3-*b*]pyridin-2-carbonsäureamid (Beispiel 4h; allgemeine Formel 4: R₁=Me, R₂=4'-NO₂-Ph-CH₂, R₃=Me, Y=S) (s. Tab. 1)

### Beispiel 9

### 9-Methyl-7-phenyl-pyrido[3',2':4,5]furo[3,2-d]pyrimidin-2,4-dion (allgemeine Formel 1a: R₁=Ph, R₂=H, R₃=Me, Y=O)

Zu 1.0 g (3.4 mmol) 3-Amino-4-methyl-6-phenyl-furo[2,3-*b*]pyridin-2-carbonsäureethylester (allgemeine Formel 8: R₁=Ph, R₂=H, R₃=Me, Y=O)* in 20 ml abs. Aceton werden 1.0 g (6.8 mmol) Benzoylisocyanat gegeben und 30 Minuten bei Raumtemperatur gerührt. Das Lösungsmittel wird entfernt und der nach Zugabe von Wasser ausgefallene Niederschlag abgesaugt und trocken gesaugt. Der Niederschlag wird in 6.8 ml (6.8 mmol) Natriumhydroxid-Lösung (1M) und 20 ml Ethanol 15 Minuten unter Rückfluss erhitzt. Das Lösungsmittel wird entfernt und der Rückstand mit 100 ml abs. Dichlormethan aufgenommen. Nach Zugabe von 0.44 ml (5.1 mmol) Oxalylchlorid wird 1 Stunde bei Raumtemperatur gerührt. Das Lösungsmittel wird entfernt und der Rückstand mit Wasser suspendiert. Der entstandene Niederschlag wird abgesaugt, mit wenig Ethanol gewaschen, trocken gesaugt und i. Vak. bei 50 °C getrocknet.
Ausbeute: 0.85 g (85 %); HPLC t_{R}: 15.0 min, Reinheit: 85.1 % (s. a in Tab. 1);
MS (ESI, m/e): 294 [M+H]⁺
(* Lit.: *Wagner G., Prantz J. Pharmazie (1990); 45, 213-214)*

### Beispiel 10

### 2-Ethyl-9-methyl-7-phenyl-pyrido[3',2':4,5]thieno[3,2-d]pyrimidin-4-on (allgemeine Formel 1b: R₁=Ph, R₂=H, R₃=Me, R₄=Et, Y=S)

Es werden 0.86 g (3.0 mmol) 3-Amino-6-pherlyl-4-methyl-thieno[2,3-b]pyridin-2-carbonsäureamid (Beispiel 4d; allgemeine Formel 4: R₁=Ph, R₂=H, R₃=Me, Y=S) und 5.0 ml (39.2 mmol) Propionsäureanhydrid in 20 ml Toluol 2 Stunden unter Rückfluß erhitzt. Nach Abkühlen wird der Niederschlag abgesaugt, mit ca. 5 ml Ethanol gewaschen und trocken gesaugt. Der Rückstand wird in 5 ml (15.0 mmol) Natriumhydroxid-Lösung (3N) 15 Minuten unter Rückfluß erhitzt. Nach Abkühlen werden 5 ml Wasser zugegeben und es wird mit Essigsäure (10 %) neutralisiert. Anschließend wird der Niederschlag abgesaugt, mit ca. 10 ml Wasser gewaschen, trocken gesaugt und i. Vak. bei 50 °C getrocknet.
Ausbeute: 0.29 g (30 %); HPLC t_{R}: 17.6 min, Reinheit: 96.2 % (s. a in Tab. 2);
MS (ESI, m/e): 322 [M+H]⁺

### Beispiel 11

### 9-Methyl-7-phenyl-2-propyl-pyrido[3',2':4,5]thieno[3,2-d]pyrimidin-4-on (allgemeine Formel 1b: R₁=Ph, R₂=H, R₃=Me, R₄=nPr, Y=S)

Analog Beispiel 10 aus 3-Amino-6-phenyl-4-methyl-thieno[2,3-*b*]pyridin-2-carbonsäureamid (Beispiel 4d; allgemeine Formel 4: R₁=Ph, R₂=H, R₃=Me, Y=S) und Buttersäureanhydrid
Es wird bei 170 °C gerührt. Nach Abkühlen wird mit Natriumhydroxid alkalisiert und kurz erwärmt. Der Niederschlag wird abgesaugt, mit Wasser gewaschen, trocken gesaugt und i. Vak. bei 50 °C getrocknet.
Ausbeute: 0.55 g (91 %); HPLC t_{R}: 20.3 min, Reinheit: 46.8 % (s. a in Tab. 2);
MS (ESI, m/e): 336 [M+H]⁺

### Beispiel 12

### 7-(3,4-Dimethoxyphenyl)-9-methyl-2-propyl-pyrido[3',2':4,5]thieno[3,2-d]pyrimidin-4-on (allgemeine Formel 1b: R₁=3,4-(MeO)₂-Ph, R₂=H, R₃=Me, R₄=nPr, Y=S)

Analog Beispiel 10 aus 3-Amino-6-(3,4-dimethoxyphenyl)-4-methyl-thieno[2,3-*b*]pyridin-2-carbonsäureamid (Beispiel 4a; allgemeine Formel 4: R₁=3,4-(MeO)₂-Ph, R₂=H, R₃=Me, Y=S) und Buttersäureanhydrid
Der Rückstand wird in 5 ml (15.0 mmol) Natriumhydroxid-Lösung (3N) und 5 ml Ethanol 6 Stunden unter Rückfluß erhitzt. Nach Ansäuern mit Salzsäure (10 %) wird der Niederschlag mit Wasser gewaschen, trocken gesaugt und i. Vak. bei 50 °C getrocknet.
Ausbeute: 0.59 g (47 %); HPLC t_{R}: 17.4 min, Reinheit: 89.8 % (s. a in Tab. 2);
MS (ESI, m/e): 396 [M+H]⁺

### Beispiel 13

### 2-Ethylthio-9-methyl-7-phenyl-pyrido[3',2':4,5]thieno[3,2-d]pyrimidin-4-on (allgemeine Formel 1b: R₁=Ph, R₂=H, R₃=Me, R₄=SEt, Y=S)

Zu 2.00 g (4.2 mmol) 3-(3-Benzoylthioureido)-4-methyl-6-phenyl-thieno[2,3-b]pyridin-2-carbonsäureethylester (Beispiel 9a; allgemeine Formel 9: R₁=Ph, R₂=H, R₃=Me, Y=S) in 25 ml Ethanol werden 10.5 ml (10.5 mmol) Natriumhydroxid-Lösung (1N) gegeben und 30 Minuten unter Rückfluß erhitzt. Nach Zugabe von 10 ml *N*,*N-*Dimethylformamid, 5 ml (5 mmol) Natriumhydroxid-Lösung (1N) und 0,50 ml lodethan (6.2 mmol) wird 30 Minuten bei Raumtemperatur gerührt. Der nach Ansäuern mit verd. Salzsäure ausgefallene Niedeschlag wird abgesaugt, mit Wasser gewaschen, trocken gesaugt und i. Vak. bei 50 °C getrocknet.
Ausbeute: 1.23 g (83 %), HPLC t_{R}: 18.5 min, Reinheit: 98.5 % (s. a in Tab. 2);
MS (ESI, m/e): 354 [M+H]⁺

**Tab. 1 Beispiele der allgemeinen Formel 1a (Y=S bei Verb. 1-8; Y=O bei Verb. 9)**

| Beispiel | R₁ | R₂ | R₃ | M [g/mol] | t_{R}^{a} [min] | Reinheit^{a} [%] | MS (m/z) |
|---|---|---|---|---|---|---|---|
| 1 | 3,4-(MeO)₂-Ph | H | Me | 369.39 | 13.8 | 99.9 | 368 [M-H]⁺ |
| 2 | 4-MeO-Ph | H | Me | 339.37 | 14.8 | 96.8 | 340 [M+H]⁺ |
| 3 | 4-F-Ph | H | Me | 327.33 | 15.4 | 99.8 | 328 [M+H]⁺ |
| 4 | Ph | H | Me | 309.34 | 14.2 | 98.3 | 310 [M+H]⁺ |
| 5 | Me | H | Ph | 309.34 | 13.0 | 99.5 | 310 [M+H]⁺ |
| 6 | Ph | H | Ph | 371.41 | 17.3 | 98.6 | 372 [M+H]⁺ |
| 7^{b} | Me | H | Me | 247.27 | 9.3 | 95.2 | 248 [M+H]⁺ |
| 8 | Me | 4'-NO₂- Ph-CH₂ | Me | 382.39 | 13.9 | 97.2 | 383 [M+H]⁺ |
| 9^{c} | Ph | H | Me | 293.28 | 15.0 | 85.1 | 294 [M+H]⁺ |

| | | | | | | | |
|---|---|---|---|---|---|---|---|
| a Retentionszeit (t_{R}) und Reinheit durch HPLC bestimmt: Säule: RP-18 (Waters Nova-Pak C₁₈, 60 Angström, 4µm, 3.9x150mm); Eluent A: 0.1 % TFA, B: 0.09% TFA /80% Acetonitril; Flussrate: 1 ml/min; Temp.: 25°C; Detektion: 254nm; Gradient (linear) B: 0-2min: 0%, 2-20min: 0-100%, 20-25min: 100%, 25-35min: 100-0% | | | | | | | |
| b Lit.: *Wagner G., Böhm N. Pharmazie (1993); 48, 95*-*99* | | | | | | | |
| c Beispiel für Verbindungen der allgemeinen Formel 1a mit Y=O | | | | | | | |

**Tab. 2 Beispiele der allgemeinen Formel 1b (Y=S)**

| Beispiel | R₁ | R₂ | R₃ | R₄ | M [g/mol] | t_{R}^{a} [min] | Reinheit^{a} [%] | MS (m/z) |
|---|---|---|---|---|---|---|---|---|
| 10 | Ph | H | Me | Et | 321.40 | 17.6 | 96.2 | 322 322 [M+H]⁺ |
| 11 | Ph | H | Me | *n*Pr | 335.42 | 20.3 | 46.8 | 336 [M+H]⁺ |
| 12 | 3,4-(MeO)₂-Ph | H | Me | *n*Pr | 395.47 | 17.4 | 89.8 | 396 [M+H]⁺ |
| 13 | Ph | H | Me | Set | 353.46 | 18.5 | 98.5 | 354 [M+H]⁺ |

| | | | | | | | | |
|---|---|---|---|---|---|---|---|---|
| a Retentionszeit (t_{R}) und Reinheit durch HPLC bestimmt: Säule: RP-18 (Waters Nova-Pak C₁₈, 60 Angström, 4pm, 3.9x150mm); Eluent A: 0.1 % TFA, B: 0.09% TFA / 80% Acetonitril; Flussrate: 1 mvmin; Temp.: 25°C; Detektion: 254nm; Gradient (linear) B: 0-2min: 0%, 2-20min: 0-100%, 20-25min: 100%, 25-35min: 100-0% | | | | | | | | |

### Beispiel 14

### Beispiele zur Herstellung von Verbindungen der allgemeinen Formel 2

### Methode A: Darstellung von 5-unsubstituierten Verbindungen der allgemeinen Formel 2

### 2-Mercapto-6-(3,4-dimethoxyphenyl)-4-methyl-pyridin-3-carbonitril (Experiment 2a; allgemeine Formel 2: R₁=3,4-(MeO)₂-Ph, R₂=H, R₃=Me, Y=S)

5.0 g (22.5 mmol) 1-(3,4-Dimethoxyphenyl)butan-1,3-dion*, 2.9 g (29.2 mmol) Cyanthio-acetamid und 4.0 g (29.2 mmol) Kaliumcarbonat werden bei Raumtemperatur 21 Stunden in 170 ml Aceton gerührt. Anschließend wird das Lösungsmittel entfernt und der Rückstand in Ethanol/Wasser (1:1) gerade gelöst. Nach Ansäuern mit Eisessig wird der erhaltene Niederschlag abgesaugt, mit wenig Wasser gewaschen, trocken gesaugt und i. Vak. bei 50 °C getrocknet.
Ausbeute: 6.34 g (98 %); HPLC t_{R}: 12.9 min, Reinheit: 87.5 % (s. a in Tab. 3);
MS (ESI, m/e): 287 [M+H]⁺
(* Nicht kommerziell verfügbare1,3-Diketone werden nach bekannten Verfahren erhalten und sind in der Fachliteratur beschrieben.)

### Methode B: Darstellung von 5-Benzyl-substituierten Verbindungen der allgemeinen Formel 2

### 4,6-Dimethyl-2-mercapto-5-(4'-nitrobenzyl)-pyridin-3-carbonitril (Experiment 2h; allgemeine Formel 2: R₁=Me, R₂=4'-NO₂-Ph-CH₂, R₃=Me, Y=S)

Zu einer Lösung von 1.28 g (55.7 mmol) Natrium in 50 ml abs. Ethanol werden 5.70 ml (55.2 mmol) Acetylaceton und 0.75 g (5.0 mmol) Natriumiodid gegeben. Nach Eintragen einer Suspension von 13.20 g (61.1 mmol) 4'-Nitrobenzylbromid in 60 ml abs. Ethanol wird 3 Stunden unter Rückfluß erhitzt. Nach Abkühlen und Filtrieren werden 5.23 g (52.2 mmol) Cyanthioacetamid und 5 ml (36.1 mmol) Triethylamin zum Filtrat hinzugefügt. Anschließend wird 4 Stunden unter Rückfluß erhitzt. Der nach Kühlen erhaltene Niederschlag wird abgesaugt, mit ca. 50 ml Ethanol und ca. 10 ml Ether rasch gewaschen, trocken gesaugt und bei i. Vak. bei 50 °C getrocknet.
Ausbeute: 5.30 g (32 %); HPLC t_{R}: 13.3 min, Reinheit: 95.9 % (s. a in Tab. 3);
MS (ESI, m/e): 300 [M+H]⁺

Analog dargestellte Beispiele der allgemeinen Formel 2 sind in Tab. 3 aufgeführt.

### Beispiel 15

### Beispiele zur Herstellung von Verbindungen der allgemeinen Formel 4

### 3-Amino-6-(3,4-dimethoxyphenyl)-4-methyl-thieno[2,3-b]pyridin-2-carbonsäureamid (Experiment 4a; allgemeine Formel 4: R₁=3,4-(MeO)₂-Ph, R₂=H, R₃=Me, Y=S)

2.1 g (31.4 mmol) Natriumethylat werden in 40 ml abs. Ethanol gelöst. Es werden 3.0 g (10.5 mmol) 2-Mercapto-6-(3,4-dimethoxyphenyl)-4-methyl-pyridin-3-carbonitril (Beispiel 2a; allgemeine Formel 2: R₁=3,4-(MeO)₂-Ph, R₂=H, R₃=Me, Y=S) und 1.2 g (12.6 mmol) 2-Chloracetamid zugegeben und 1 Stunde unter Rückfluß erhitzt. Nach dem Abkühlen werden 10 ml Wasser zugegeben und der Niederschlag abgesaugt, trocken gesaugt und i. Vak. bei 50 °C getrocknet.
Ausbeute: 3.4 g (94 %); HPLC t_{R}: 14.0 min, Reinheit: 99.8 % (s. a in Tab. 4);
MS (ESI, m/e): 344 [M+H]⁺

Analog dargestellte Beispiele der allgemeinen Formel 4 sind in Tab. 4 aufgeführt.

### Beispiel 16

### Beispiele zur Herstellung von Verbindungen der allgemeinen Formel 8

### 3-Amino-4-methyl-6-phenyl-thieno[2,3-b]pyridin-2-carbonsäureethylester (Experiment 8a; allgemeine Formel 8: R₁=Ph, R₂=H, R₃=Me, Y=S)

6.45 g (28.5 mmol) 2-Mercapto-6-phenyl-4-methyl-pyridin-3-carbonitril (Beispiel 2d; allgemeine Formel 2: R₁=Ph, R₂=H, R₃=Me, Y=S) werden in 100 ml abs. Ethanol vorgelegt und 5.3 ml (50.0 mmol) 2-Chloressigsäureethylester sowie 10 ml (72.1 mmol) Triethylamin hinzugefügt. Es wird 1 Stunde unter Rückfluß erhitzt und der nach Kühlen ausgefallene Niederschlag abgesaugt, mit wenig Wasser und Ethanol gewaschen, trocken gesaugt und i. Vak. bei 50 °C getrocknet.
Anschließend wird der Niederschlag in einer Lösung aus 0.14 g (1.6 mmol) Natriumethylat in abs. Ethanol 1 Stunde unter Rückfluß erhitzt. Der nach Abkühlen ausgefallene Niederschlag wird abgesaugt, mit 30 ml Wasser und 15 ml Ethanol gewaschen, trocken gesaugt und i. Vak. bei 50 °C getrocknet.
Ausbeute: 5.0 g (91 %); HPLC t_{R}: 18.0 min, Reinheit: 98.4 % ;
MS (ESI, m/e): 313 [M+H]⁺

### Beispiel 17

### Beispiele zur Herstellung von Verbindungen der allgemeinen Formel 9

### 3-(3-Benzoylthioureido)-4-methyl-6-phenyl-thieno[2,3-b]pyridin-2-carbonsäureethylester (Beispiel 9a; allgemeine Formel 9: R₁=Ph, R₂=H, R₃=Me, Y=S)

Zu 2.78 g (8.9 mmol) 3-Amino-4-methyl-6-phenyl-thieno[2,3-b]pyridin-2-carbonsäureethylester (Beispiel 8a; allgemeine Formel 8: R₁=Ph, R₂=H, R₃=Me, Y=S) in 10 ml Aceton werden 5 ml (13.3 mmol) acetonischer Benzoylisothiocyanat-Lösung (2M) gegeben und 2 Stunden unter Rückfluß erhitzt. Der nach Abkühlen ausgefallene Niederschlag wird abgesaugt, mit wenig Wasser und Ethanol gewaschen, trocken gesaugt und i. Vak. bei 50 °C getrocknet.
Ausbeute: 3.93 g (93 %); HPLC t_{R}: 19.3 min, Reinheit: 99.9 % (s. a in Tab. 4);
MS (ESI, m/e): 476 [M+H]⁺

**Tab. 3 Beispiele der allgemeinen Formel 2 (Y=S)**

| Exp. | R₁ | R₂ | R₃ | M [g/mol] | t_{R}^{a} [min] | Reinheit^{a} [%] | MS (m/z) |
|---|---|---|---|---|---|---|---|
| 2a | 3,4-(MeO)₂-Ph | H | Me | 286.35 | 12.9 | 87.5 | 287 [M+H]⁺ |
| 2b^{b,c} | 4-MeO-Ph | H | Me | 256.32 | 13.4 | 89.0 | 257 [M+H]⁺ |
| 2c^{b,d} | 4-F-Ph | H | Me | 244.29 | 12.2 | 89.7 | 245 [M+H]⁺ |
| 2d^{b,e} | Ph | H | Me | 226.30 | 12.0 | 87.9 | 227 [M+H]⁺ |
| 2e^{f,g} | Me | H | Ph | 226.30 | 11.7 | 52.0 | 227 [M+H]⁺ |
| 2f^{b,h} | Ph | H | Ph | 288.37 | 15.8 | 88.5 | 289 [M+H]⁺ |
| 2g^{b,i} | Me | H | Me | 164.23 | 9.1 | 35.6 | 165 [M+H]⁺ |
| 2hⁱ | Me | 4'-NO₂-Ph-CH₂ | Me | 299.35 | 13.3 | 95.5 | 300 [M+H]⁺ |

| | | | | | | | |
|---|---|---|---|---|---|---|---|
| a Retentionszeit (t_{R}) und Reinheit durch HPLC bestimmt: Säule: RP-18 (Waters Nova-Pak C₁₈, 60 Angström, 4µm, 3.9 x 150mm); Eluent A: 0.1% TFA, B: 0.09% TFA / 80% Acetonitril; Flussrate: 1 ml/min; Temp.: 25°C; Detektion: 254nm; Gradient (linear) B: 0-2min: 0%, 2-20min: 0-100%, 20-25min: 100%, 25-35min: 100-0% | | | | | | | |
| b Darstellung erfolgt nach Methode A: Erhitzen unter Rückfluß für 3-4 Stunden; nach Abkühlen wird der Ansatz ggf. filtriert und das Filtrat weiterverarbeitet | | | | | | | |
| c Lit.: *Vieweg H., Leistner S., Wagner G. Pharmazie (1986); 41, 827-830* | | | | | | | |
| d Lit.: *Vieweg H., Krasselt U., Boehm N., Prantz J., Wagner G., Pharmazie (1990); 45, 731*-*733* | | | | | | | |
| e Lit.: *Schmidt U., Kubitzek H., Chem Ber (1960); 93, 1559-1565* | | | | | | | |
| f Darstellung erfolgt nach Methode A: Erhitzen unter Rückfluß für 3-4 Stunden; nach Abkühlen wird der Niederschlag abgesaugt und analog dem Rückstand weiterverarbeitet. | | | | | | | |
| g Lit.: *Krauze A., Bomika Z., Shestopalov A.M., Rodinovskaya L.A., Pelcers J., Duburs G., Sharanin Y.A., Promonenkov V.K. Khim Geterotsikl Soedin (1981); 377-382* | | | | | | | |
| h Lit.: *Guerrera F., Siracusa M.A., Tometta B. Farmaco, Ediz Scientif (1976); 31, 21-30* | | | | | | | |
| i Lit.: *Wallenfels K., Schuly H., Ann (1959); 621, 215-221* | | | | | | | |
| j Darstellung erfolgt nach Methode B, in Anlehnung an *Wagner G., Vieweg H., Leistner S., Böhm N., Krasselt U., Hanfeld V., Prantz J., Grupe R. Pharmazie (1990); 45, 102-109* | | | | | | | |

**Tab. 4 Beispiele der allgemeinen Formel 4 (Y=S)**

| Exp. | R₁ | R₂ | R₃ | M [g/mol] | t_{R}^{a} [min] | Reinheit^{a} [%] | MS (m/z) |
|---|---|---|---|---|---|---|---|
| 4a | 3,4-(MeO)₂-Ph | H | Me | 343.40 | 14.0 | 99.8 | 344 [M+H]⁺ |
| 4b | 4-MeO-Ph | H | Me | 313.37 | 14.8 | 99.9 | 314 [M+H]⁺ |
| 4c | 4-F-Ph | H | Me | 301.34 | 15.5 | 99.5 | 302 [M+H]⁺ |
| 4d^{b} | Ph | H | Me | 283.35 | 14.8 | 99.5 | 284 [M+H]⁺ |
| 4e^{b,c} | Me | H | Ph | 283.35 | 14.2 | 99.3 | 284 [M+H]⁺ |
| 4f^{d} | Ph | H | Ph | 345.42 | 17.5 | 99.2 | 346 [M+H]⁺ |
| 4g^{e} | Me | H | Me | 221.28 | 9.5 | 98.4 | 222 [M+H]⁺ |
| 4h | Me | 4'-NO₂-Ph-CH₂ | Me | 356.40 | 13.5 | 98.2 | 357 [M+H]⁺ |

| | | | | | | | |
|---|---|---|---|---|---|---|---|
| a Retentionszeit (t_{R}) und Reinheit durch HPLC bestimmt: Säule: RP-18 (Waters Nova-Pak C₁₈, 60 Angström, 4pm, 3.9x150mm); Eluent A: 0.1% TFA, B: 0.09% TFA / 80% Acetonitril; Flussrate: 1 ml/min; Temp.: 25°C; Detektion: 254nm; Gradient (linear) B: 0-2min: 0%, 2-20min: 0-100%, 20-25min: 100%, 25-35min: 100-0% | | | | | | | |
| b Lit.: *Litvinov V.P., Sharanin Y.A., Rodinovskaya L.A., Shestopalov A.M., Mortikov V. Yu., Promonenkov V.K. Ser Khimich (1984); 12, 2760-2765* | | | | | | | |
| c Lit.: *Sharanin Y.A., ShestopalovA.M., Promonenkov V.K. Zhumal Organ Khimii (1984); 20, 2012-2020* | | | | | | | |
| d Lit.: *Shestopalov A.M., Sharanin Y.A. Zhurnal Organ Khimii (1984); 20, 1991-2002* | | | | | | | |
| e Lit.: *Tornetta B., Siracusa M.A., Ronsisvalle G., Guerrera F. Gazz. Chim. ltal (1978); 108, 57-62* und *Shvedov V.L, Sycheva T.P., Sakovich T. V. Khimiya Geterot Soedin (1979); 1331-1335* | | | | | | | |

## Patentansprüche

1. Substituierte Pyrido[3',2':4,5]thieno[3,2-d]pyrimidin-2,4(1H,3H)-dione und -4(3H)-one sowie
Pyrido[3',2':4,5]furo[3,2-d]pyrimidin-2,4(1H,3H)-dione und -4(3H)-one der allgemeinen Formeln 1a und 1b 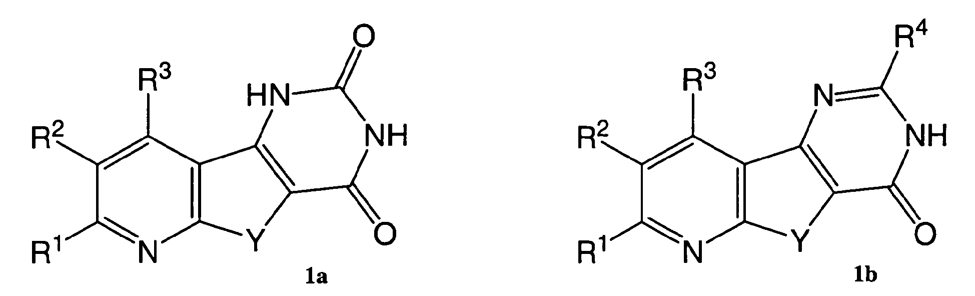
worin bedeuten:
Y Schwefel oder Sauerstoff und
R¹ und R³, gleich oder ungleich unabhängig voneinander,
- Wasserstoff,
- C₁₋₁₂Alkyl (ggf. mit R^{§} substituiert),
- C_{2-!2} Alkenyl und C_{2-!2} Alkinyl (jeweils ggf. mit R^{§} substituiert ),
- Benzyl (ggf. mit R^{§} substituiert), Phenyl-(C₂₋₆)alkyl , Phenyl, 4-R^{§}-Phenyl, 3-R^{§} -Phenyl, 2-R^{§}-Phenyl, 3-R^{§},4-R^{§}-Phenyl, 3-R^{§},4-R^{§}-,5-R^{§}-Phenyl,
- Difluormethyl, Trifluormethyl,
- 1-Naphthyl, 2-Naphthyl (jeweils ggf. mit R^{§} substituiert),
- C₃₋₁₄Cycloalkyl, C₃₋₁₄Cycloalkenyl (jeweils ggf. mit R^{§}-substituiert),
- mono- oder bicyclische gesättigte oder ein- oder mehrfach ungesättigte Heterocyclen mit 5 - 14 Ringatomen darunter 1 - 4 Heteroatome, die vorzugsweise N, O und S sind,
- C₁₋₁₂Alkylacyl (ggf. mit R^{§} substituiert),
- Benzoyl, 1- und 2-Naphthoyl (jeweils ggf. mit R^{§} substituiert),
- Heterocyclylacyl [z.B. Nicotinoyl, Isonicotinoyl, 2-Picolinoyl, 2-Thienoyl, 2-Furoyl] (ggf. mit R^{§} substituiert)
- Hydroxy,
- Sulfhydryl,
- C₁₋₁₀ Alkoxy,
- Alkylthio, Alkylsulfinyl und Alkylsulfonyl (jeweils C₁₋₆)
- Formyl, Carboxyl, C₁₋₄Alkoxycarbonyl;
- CONH₂, CONHAlk und CONAIk₂ (mit "Alk" jeweils C₁₋₆),
- Cyano, Rhodano, Nitro, SO₃H, SO₂OAlk (mit "Alk": C₁₋₅),
- Chlor, Brom, lod, Fluor,
- Amino, C₁₋₆Alkylamino, Di(C₁₋₅)alkylamino (jeweils ggf. mit R^{§} am Alkylrest substituiert),
- Morpholino, Thiomorpholino, Thiomorpholino-S,S-Dioxid, Pyrrolidino,
- Piperidino, 1-Piperazino, 4-Methyl-1-piperazino, 4-Hydroxyethyl-1-piperazino, 4-Phenyl-1-piperazino,
- Cycloalkylamino, C₃₋₁₄ Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo- bzw. heterocyclischen Ringen mit R^{§} substituiert);
R²
- Wasserstoff,
- C₁₋₁₂ Alkyl,
- C₂₋₁₂ Alkenyl und C₂₋₁₂ Alkinyl,
- Benzyl, Phenyl(C₂₋₆)alkyl (ggf. mit R^{§} ein oder mehrmals, gleich oder ungleich am aromatischen und/oder aliphatischen Molekülteil substituiert);
- Phenacyl (ggf. mit R^{§} ein oder mehrmals, gleich oder ungleich am aromatischen Molekülteil substituiert);
- Carboxyl, C₁₋₄ Alkoxycarbonyl, CONH₂, CONHAIk und CONAlk₂ (mit "Alk" jeweils C₁-6),
- R*CO- (worin R* Wasserstoff, C₁-C₁₂Alkyl bedeuten sowie ggf. mit R^{§} substituiert),
- Cyano, Nitro, Amino, C₁₋₆Alkylamino, Di(C₁₋₆)alkylamino, -N=N-C₆H₅, -N=N-C₆H₄-R^{§},
- 1,3-Diphenyl-pyrazol-4-yl, Thiazolin-2-yl, Imidazolin-2-yl und 3,4,5,6-Tetrahydro-pyrimidinyl;
R⁴
- C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten aliphatischen oder cycloaliphatischen Reste mit R^{§} substituiert);
- Phenyl, 2-R^{§}-Phenyl, 3-R^{§}-Phenyl, 4-R^{§}-Phenyl, 1-R^{§},5-R^{§}-Phenyl, 3-R^{§},5-R^{§}-,5-R^{§}-Phenyl, 3-R^{§},4-R^{§}-Phenyl, 3-R^{§},4-R^{§}-,5-R^{§}-Phenyl, 1-R^{§},3-R^{§}-4-R^{§}-Phenyl
- 1-Naphthyl, 2-Naphthyl (jeweils ggf. mit R^{§} substituiert);
- mono-, bi- oder tricyclische gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert);
- CH₂OAlk* (mit Alk*: C₁₋₆Alkyl),
- CH₂OCOR' (mit R': C₁₋₆Alkyl, C₂₋₆Alkenyl, Phenyl sowie mit R^{§} substituiert),
- COOH, COOAlk** (mit Alk**: C₁₋₅Alkyl),
- NH₂, NHAlk*, (mit Alk*: C₁₋₆Alkyl)
- NHNH₂, NHNHCOR' (mit R': C₁₋₆Alkyl, C₁₋₆Alkenyl, Phenyl sowie mit R^{§} substituiert),
- SO₃H, S-Phenacyl, S-Alkyl, SO-Alkyl, SO₂-Alkyl (jeweils C₁-C₈) S-Alkenyl, SO-Alkenyl, SO₂-Alkenyl (jeweils C₂-C₈) S-Alkinyl, SO-Alkinyl, SO₂-Alkinyl (jeweils C₂-C₆) (ggf. jeweils am C-Skelett der oben genannten aliphatischen Reste mit -OH, -CN, -SCN, -NO₂, Phenyl oder (C₃-C₇)Cycloalkyl substituiert)
R^{§} -OH, -SH, -O-C₁₋₈Alkyl, -O-C₆₋₁₄Aryl, -S-C₁₋₄ Alkyl, -S-C₆₋₁₄Aryl, -SO-C₁₋₄Alkyl, -SO-C₆₋₁₄Aryl, -SO₂-C₁₋₄Alkyl, -SO₂-C₆₋₁₄Aryl, -SO₃H, -OSO₂C₁₋₈Alkyl, -OSO₂C₆₋₁₄Aryl, -COOH, -COOC₁₋₈Alkyl, -(CO)C₁₋₈Alkyl, -COOH, -COOC_{1...8}Alkyl, -CONH₂, -CONHC₁₋₆Alkyl, -CON(C₁₋₆Alkyl)₂, -NH₂, -NHC₁₋₆Alkyl, -N(C₁₋₆Alkyl)₂, -NHC₆₋₁₄Aryl, -NH-Hetaryl, -N(C₆₋₁₄Aryl)₂, -N(C₁₋₆Alkyl)(C₆₋₁₄Aryl),
-CH₃, -CHF₂, -CF₃, -C₂H₅, -C(CH₃)₂, -(CH₂)₂CH₃, -(CH₂)₃CH₃, -CH₂CH₂OH, -CH₂CH₂SH, -CH₂CH₂SCH₃, -CH₂CF₃, -CH₂CCl₃, -CH₂CHF₂, -CH₂CHCl₂, -CH₂CH₂F, -CH₂CH₂Cl, -CH₂CH₂Br, -Cylopropyl, -Cylopropylmethyl, -Cylobutyl, -Cyclobutylmethyl, -Cyclopentyl, -Cyclopentylmethyl, -Cylohexyl, -Cyclohexylmethyl,
-F, -Cl, -Br, -I, -CN, -NO₂, und -SCN.
sowie deren Solvate, einschließlich der pharmazeutisch akzeptablen Salze und Prodrug-Formulierungen und deren aktive Metabolite und gegebenenfalls deren Tautomere
wobei die folgenden Verbindungen der Formel 1a ausgenommen sind:
| | R¹ | R² | R³ |
|---|---|---|---|
| 1. | Me | H | Me |
| 2. | Me | Ac | 4-NMe₂Ph |
| 3. | Me | Ac | 4-NO₂Ph |
wobei die die folgenden Verbindungen der Formel 1b mit folgenden Resten ausgenommen sind:
jeweils R¹ = OEt, R² = CN, R³ = Ph mit R⁴ = Benzyl, Phenyl, z.T. mehrfach mit Me, OMe, NO₂ und Cl substituiert und 3,4-Methylendioxy-phenyl;
jeweils R¹ = Ph, R² = H, R³ = Ph mit R⁴ = Phenyl, p-OCH₃-Ph, p-Cl-Ph, 2,3,4-Tri-OCH₃-Ph und 2-Thienyl;
jeweils R¹ = Me, R² = H, R³ = Me mit R⁴ = CH₂OMe, CH₂OEt, CH₂OCOMe, CH₂COOPh, SCH₂COPh, NH₂, NHNH₂, COOH, Ph; sowie:
R¹ = Me, R² = H, R³ = Ph, R⁴ = COOEt;
R¹ = Me, R² = H, R³ = p-Br-Ph, R⁴ = COOEt;
R¹ = p-Br-Ph, R² = H, R³ = Me, R⁴ = COOMe;
R¹ = Me, R² = Benzyl, R³ = Me, R⁴ = COOMe;
R¹ = R² = R³ = Me, R⁴ = COOMe.

2. Verbindung gemäß Anspruch 1, wobei diese Formel 1a mit Y = S entspricht und die Substituenten R₁, R₂, R₃ die folgenden Bedeutungen haben:
| R₁ | R₂ | R₃ |
|---|---|---|
| 3,4-(MeO)₂-Ph | H | Me |
| 4-MeO-Ph | H | Me |
| 4-F-Ph | H | Me |
| Ph | H | Me |
| Me | H | Ph |
| Ph | H | Ph |
| Me | H | Me |
| Me | 4'-NO₂-Ph-CH₂ | Me |

3. Verbindung gemäß Anspruch 1, wobei diese Formel 1a mit Y = O entspricht und R₁ = Ph, R₂= H und R₃ = Me.

4. Verbindung gemäß Anspruch 1, wobei diese Formel 1b mit Y = S entspricht und die Substituenten R₁ , R₂ , R₃ ,R₄ die folgenden Bedeutungen haben:
| R₁ | R₂ | R₃ | R₄ |
|---|---|---|---|
| Ph | H | Me | Et |
| Ph | H | Me | *n*Pr |
| 3,4-(MeO)₂-Ph | H | Me | *n*Pr |
| Ph | H | Me | Set |

5. Verbindung gemäß Anspruch 1, wobei diese ausgewählt ist aus:
2-Ethyl-9-methyl-7-phenyl-pyrido[3',2':4,5]thieno[3,2-d]pyrimidin-4(3H)-on;
7-(3,4-Dimethoxy)-9-methyl-pyrido[3',2':4,5]thieno[3,2-d]pyrimidin-2,4(1H,3H)-dion;
7-Phenyl-2-n-propyl-9-(pyrid-3-yl)-pyrido[3',2':4,5]thieno[3,2-d]pyrimidin-4(3H)-on;
7-(4-Fluor-phenyl)-9-methyl-pyrido[3',2':4,5]thieno[3,2-d]pyrimidin-2,4(1H,3H)-dion;
7-(4-Methoxy-phenyl)-9-methyl-pyrido[3',2':4,5]thieno[3,2-d]pyrimidin-2,4(1H,3H)-dion;
7-Phenyl-pyrido[3',2':4,5]thieno[3,2-d]pyrimidin-4(3H)-on-2-carbonsäure;
9-Methyl-7-phenyl-pyrido[3',2':4,5]thieno[3,2-d]pyrimidin-2,4(1H,3H)-dion;
7-Phenyl-pyrido[3',2':4,5]thieno[3,2-d]pyrimidin-4(3H)-on-2-carbonsäure;
7,9-Dimethyl-pyrido[3',2':4,5]thieno[3,2-d]pyrimidin-2,4(1H,3H)-dion;

6. Pharmazeutische Zusammensetzung enthaltend mindestens eine der nachfolgenden Verbindungen der Formel 1a oder 1b sowie ggf. pharmazeutisch verträgliche Hilfsstoffe und/oder Träger: 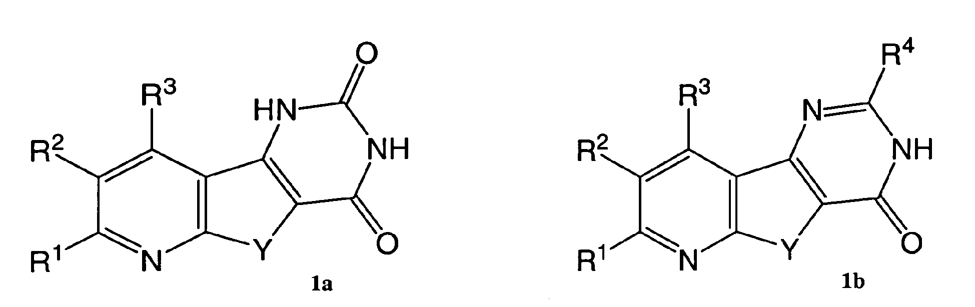
worin bedeuten:
Y Schwefel oder Sauerstoff und
R¹ und R³, gleich oder ungleich unabhängig voneinander,
- Wasserstoff,
- C₁₋₁₂Alkyl (ggf. mit R^{§} substituiert),
- C₂₋₁₂ Alkenyl und C₂₋₁₂ Alkinyl (jeweils ggf. mit R^{§} substituiert),
- Benzyl (ggf. mit R^{§} substituiert), Phenyl-(C₂₋₆)alkyl , Phenyl, 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl,
- Difluormethyl, Trifluormethyl,
- 1-Naphthyl, 2-Naphthyl (jeweils ggf. mit R^{§} substituiert),
- C₃₋₁₄Cycloalkyl, C₃₋₁₄Cycloalkenyl (jeweils ggf. mit R^{§}-substituiert),
- mono- oder bicyclische gesättigte oder ein- oder mehrfach ungesättigte Heterocyclen mit 5 - 14 Ringatomen darunter 1 - 4 Heteroatome, die vorzugsweise N, O und S sind,
- C₁₋₁₂Alkylacyl (ggf. mit R^{§} substituiert),
- Benzoyl, 1- und 2-Naphthoyl (jeweils ggf. mit R^{§} substituiert),
- Heterocyclylacyl [z.B. Nicotinoyl, Isonicotinoyl, 2-Picolinoyl, 2-Thienoyl, 2-Furoyl] (ggf. mit R^{§} substituiert)
- Hydroxy,
- Sulfhydryl,
- C₁₋₁₀ Alkoxy,
- Alkylthio, Alkylsulfinyl und Alkylsulfonyl (jeweils C₁₋₆)
- Formyl, Carboxyl, C₁₋₄Alkoxycarbonyl;
- CONH₂, CONHAlk und CONAlk₂ (mit "Alk" jeweils C₁₋₆),
- Cyano, Rhodano, Nitro, SO₃H, SO₂OAlk (mit "Alk": C₁₋₅),
- Chlor, Brom, lod, Fluor,
- Amino, C₁₋₆Alkylamino, Di(C₁₋₅)alkylamino (jeweils ggf. mit R^{§} am Alkylrest substituiert),
- Morpholino, Thiomorpholino, Thiomorpholino-S,S-Dioxid, Pyrrolidino,
- Piperidino, 1-Piperazino, 4-Methyl-1-piperazino, 4-Hydroxyethyl-1-piperazino, 4-Phenyl-1-piperazino,
- Cycloalkylamino, C₃₋₁₄ Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo- bzw. heterocyclischen Ringen mit R^{§} substituiert);
R²
- Wasserstoff,
- C₁₋₁₂ Alkyl,
- C₂₋₁₂ Alkenyl und C₂₋₁₂ Alkinyl,
- Benzyl, Phenyl(C₂₋₆)alkyl (ggf. mit R^{§} ein oder mehrmals, gleich oder ungleich am aromatischen und/oder aliphatischen Molekülteil substituiert);
- Phenacyl (ggf. mit R^{§} ein oder mehrmals, gleich oder ungleich am aromatischen Molekülteil substituiert);
- Carboxyl, C₁₋₄ Alkoxycarbonyl, CONH₂, CONHAlk und CONAlk₂ (mit "Alk" jeweils C₁-6),
- R*CO- (worin R* Wasserstoff, C₁-C₁₂Alkyl bedeuten sowie ggf. mit R^{§} substituiert),
- Cyano, Nitro, Amino, C₁₋₆Alkylamino, Di (C₁₋₆)alkylamino, -N=N-C₆H₅, -N=N-C₆H₄-R^{§},
- 1,3-Diphenyl-pyrazol-4-yl, Thiazolin-2-yl, Imidazolin-2-yl und 3,4,5,6-Tetrahydro-pyrimidinyl;
R⁴
- C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten aliphatischen oder cycloaliphatischen Reste mit R^{§} substituiert);
- Phenyl, 2-R^{§}-Phenyl, 3-R^{§}-Phenyl, 4-R^{§}-Phenyl, 1-R^{§},5-R^{§}-Phenyl, 3-R^{§},5-R^{§}-,5-R^{§}-Phenyl, 3-R^{§},4-R^{§}-Phenyl, 3-R^{§},4-R^{§}-,5-R^{§}-Phenyl, 1-R^{§},3-R^{§}-4-R^{§}-Phenyl
- 1-Naphthyl, 2-Naphthyl (jeweils ggf. mit R^{§} substituiert);
- mono-, bi- oder tricyclische gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert);
- CH₂OAlk* (mit Alk*: C₁₋₆Alkyl),
- CH₂OCOR' (mit R': C₁₋₆Alkyl, C₂₋₆Alkenyl, Phenyl sowie mit R^{§} substituiert),
- COOH, COOAlk** (mit Alk**: C₁₋₅Alkyl),
- NH₂, NHAlk*, (mit Alk*: C₁₋₆Alkyl)
- NHNH₂, NHNHCOR' (mit R': C₁₋₆Alkyl, C₁₋₆Alkenyl, Phenyl sowie mit R^{§} substituiert),
- SO₃H, S-Phenacyl, S-Alkyl, SO-Alkyl, SO₂-Alkyl (jeweils C₁-C₈) S-Alkenyl, SO-Alkenyl, SO₂-Alkenyl (jeweils C₂-C₈) S-Alkinyl, SO-Alkinyl, SO₂-Alkinyl (jeweils C₂-C₆)
(ggf. jeweils am C-Skelett der oben genannten aliphatischen Reste mit -OH, -CN, -SCN, -NO₂, Phenyl oder (C₃-C₇)Cycloalkyl substituiert)
R^{§} -OH, -SH, -O-C₁₋₈Alkyl, -O-C₆₋₁₄ Aryl, -S-C₁₋₄ Alkyl, -S-C₆₋₁₄Aryl, -SO-C₁₋₄Alkyl, -SO-C₆₋₁₄Aryl, -SO₂-C₁₋₄Alkyl, -SO₂-C₆₋₁₄Aryl, -SO₃H, -OSO₂C₁₋₈Alkyl, -OSO₂C₆₋₁₄Aryl, -COOH, -COOC₁₋₈Alkyl, -(CO)C₁₋₈Alkyl, -COOH, -COOC₁₋₈Alkyl, -CONH₂, -CONHC₁₋₆Alkyl, -CON(C₁₋₆Alkyl)₂, -NH₂, -NHC₁₋₆Alkyl, -N(C₁₋₆Alkyl)₂, -NHC₆₋₁₄Aryl, -NH-Hetaryl, -N(C₆₋₁₄Aryl)₂, -N(C₁₋₆Alkyl)(C₆₋₁₄Aryl),
-CH₃, -CHF₂, -CF₃, -C₂H₅, -C(CH₃)₂, -(CH₂)₂CH₃, -(CH₂)₃CH₃, -CH₂CH₂OH, -CH₂CH₂SH, -CH₂CH₂SCH₃, -CH₂CF₃, -CH₂CCl₃, -CH₂CHF₂, -CH₂CHCl₂, -CH₂CH₂F, -CH₂CH₂Cl, -CH₂CH₂Br, -Cylopropyl, -Cylopropylmethyl, -Cylobutyl, -Cyclobutylmethyl, -Cyclopentyl, -Cyclopentylmethyl, -Cylohexyl, -Cyclohexylmethyl,
-F, -CI, -Br, -1, -CN, -NO₂, und -SCN.
sowie deren Solvate, einschließlich der pharmazeutisch akzeptablen Salze und Prodrug-Formulierungen und deren aktive Metabolite und gegebenenfalls deren Tautomere.

7. Pharmazeutische Zusammensetzung nach Anspruch 6 enthaltend zusätzlich einen oder mehrere der folgenden Wirkstoffe:
- Zytokin-Antagonisten
- Chemokin-Antagonisten
- Zytokin-Agonisten
- immunmodulatorische Wirkstoffe
- Substanz P-Antagonisten
- Bradykinin-Antagonisten
- PAF-Antagonisten
- Adenosin-Rezeptor-Antagonisten
- Antibiotika/Virostatika
- α-Mimetika
- Zytostatika
- β₂-Adrenoceptor Agonisten
- Leukotrien-Antagonisten (entweder Enzym-Inhibitoren [wie 5-Lipoxygenaseinhibitoren oder Arachidonsäure-Enzyminhibitoren] oder Rezeptorantagonisten),
- Antihistaminika (bevorzugt solche mit Mastzellen-stabilisierenden Eigenschaften oder Leukotrien-antagonisierenden Aspekten)
- Theophyllin
- Muscarinrezeptor-Antagonisten

8. Verwendung von mindestens eine der nachfolgenden Verbindungen der Formel 1a oder 1b sowie ggf. pharmazeutisch verträgliche Hilfsstoffe und/oder Träger. 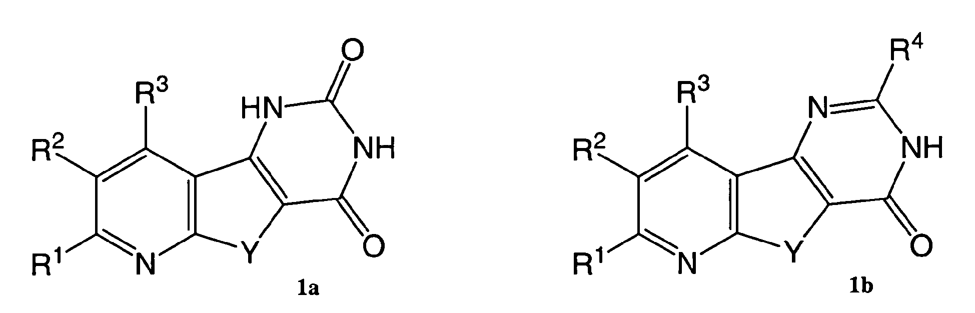
worin bedeuten:
Y Schwefel oder Sauerstoff und
R¹ und R³, gleich oder ungleich unabhängig voneinander,
- Wasserstoff,
- C₁₋₁₂Alkyl (ggf. mit R^{§} substituiert),
- C_{2-!2} Alkenyl und C₂-_{!2} Alkinyl (jeweils ggf. mit R^{§} substituiert),
- Benzyl (ggf. mit R^{§} substituiert), Phenyl-(C₂₋₆)alkyl , Phenyl, 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl,
- Difluormethyl, Trifluormethyl,
- 1-Naphthyl, 2-Naphthyl (jeweils ggf. mit R^{§} substituiert),
- C₃-₁₄Cycloalkyl, C₃-₁₄Cycloalkenyl (jeweils ggf. mit R^{§}-substituiert),
- mono- oder bicyclische gesättigte oder ein- oder mehrfach ungesättigte Heterocyclen mit 5 - 14 Ringatomen darunter 1 - 4 Heteroatome, die vorzugsweise N, O und S sind,
- C₁₋₁₂Alkylacyl (ggf. mit R^{§} substituiert),
- Benzoyl, 1- und 2-Naphthoyl (jeweils ggf. mit R^{§} substituiert),
- Heterocyclylacyl [z.B. Nicotinoyl, Isonicotinoyl, 2-Picolinoyl, 2-Thienoyl, 2-Furoyl] (ggf. mit R^{§} substituiert)
- Hydroxy,
- Sulfhydryl,
- C₁₋₁₀ Alkoxy,
- Alkylthio, Alkylsulfinyl und Alkylsulfonyl (jeweils C₁₋₆)
- Formyl, Carboxyl, C₁₋₄Alkoxycarbonyl;
- CONH₂, CONHAIk und CONAIk₂ (mit "Alk" jeweils C₁₋₆),
- Cyano, Rhodano, Nitro, SO₃H, SO₂OAlk (mit "Alk": C₁₋₅),
- Chlor, Brom, lod, Fluor,
- Amino, C₁₋₆Alkylamino, Di(C₁₋₅)alkylamino (jeweils ggf. mit R^{§} am Alkylrest substituiert),
- Morpholino, Thiomorpholino, Thiomorpholino-S,S-Dioxid, Pyrrolidino,
- Piperidino, 1-Piperazino, 4-Methyl-1-piperazino, 4-Hydroxyethyl-1-piperazino, 4-Phenyl-1-piperazino,
- Cycloalkylamino, C₃₋₁₄ Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo- bzw. heterocyclischen Ringen mit R^{§} substituiert);
R²
- Wasserstoff,
- C₁₋₁₂ Alkyl,
- C₂₋₁₂ Alkenyl und C₂₋₁₂ Alkinyl,
- Benzyl, Phenyl(C₂₋₆)alkyl (ggf. mit R^{§} ein oder mehrmals, gleich oder ungleich am aromatischen und/oder aliphatischen Molekülteil substituiert);
- Phenacyl (ggf. mit R^{§} ein oder mehrmals, gleich oder ungleich am aromatischen Molekülteil substituiert);
- Carboxyl, C₁₋₄ Alkoxycarbonyl, CONH₂, CONHAlk und CONAlk₂ (mit "Alk" jeweils C₁₋₆),
- R*CO- (worin R* Wasserstoff, C₁-₁₂Alkyl bedeuten sowie ggf. mit R^{§} substituiert),
- Cyano, Nitro, Amino, C₁₋₆Alkylamino, Di(C₁₋₆)alkylamino, -N=N-C₆H₅, -N=N-C₆H₄-R^{§},
- 1,3-Diphenyl-pyrazol-4-yl, Thiazolin-2-yl, Imidazolin-2-yl und 3,4,5,6-Tetrahydro-pyrimidinyl;
R⁴
- C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten aliphatischen oder cycloaliphatischen Reste mit R^{§} substituiert);
- Phenyl, 2-R^{§}-Phenyl, 3-R^{§}-Phenyl, 4-R^{§}-Phenyl, 2-R^{§},5-R^{§}-Phenyl, 3-R^{§},5-R^{§}-Phenyl, 3-R^{§},4-R^{§}-Phenyl, 3-R^{§},4-R ,5-R^{§}-Phenyl, 2-R^{§},3-R^{§},4-R^{§}-Phenyl,
- 1-Naphthyl, 2-Naphthyl (jeweils ggf. mit R^{§} substituiert);
- mono-, bi- oder tricyclische gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert);
- CH₂OAlk* (mit Alk*: C₁₋₆Alkyl),
- CH₂OCOR' (mit R': C₁₋₆Alkyl, C₂₋₆Alkenyl, Phenyl sowie mit R^{§} substituiert),
- COOH, COOAlk** (mit Alk**: C₁₋₅Alkyl),
- NH₂, NHAlk*, (mit Alk*: C₁₋₆Alkyl)
- NHNH₂, NHNHCOR' (mit R': C₁₋₆Alkyl, C₁₋₆Alkenyl, Phenyl sowie mit R^{§} substituiert),
- SO₃H, S-Phenacyl, S-Alkyl, SO-Alkyl, SO₂-Alkyl (jeweils C₁-₈) S-Alkenyl, SO-Alkenyl, SO₂-Alkenyl (jeweils C₂-₈) S-Alkinyl, SO-Alkinyl, SO₂-Alkinyl (jeweils C₂-₆)
(ggf. jeweils am C-Skelett der oben genannten aliphatischen Reste mit -OH, -CN, -SCN, -NO₂, Phenyl oder (C₃-₇)Cycloalkyl substituiert)
R^{§} -OH, -SH, -O-C₁₋₈Alkyl, -O-C₆₋₁₄ Aryl, -S-C₁₋₄Alkyl, -S-C₆₋₁₄Aryl, -SO-C₁₋₄Alkyl, -SO-C₆₋₁₄Aryl, -SO₂-C₁₋₄Alkyl, -SO₂-C₆₋₁₄Aryl, -SO₃H, -OSO₂C₁₋₈Alkyl, -OSO₂C₆₋₁₄Aryl, -COOH, -COOC₁₋₈Alkyl, -(CO)C₁₋₈Alkyl, -COOH, -COOC₁₋₈Alkyl, -CONH₂, -CONHC₁₋₆Alkyl, -CON(C₁₋₆Alkyl)₂, -NH₂, -NHC₁₋₆Alkyl, -N(C₁₋₆Alkyl)₂, -NHC₆₋₁₄Aryl, -NH-Hetaryl, -N(C₆₋₁₄Aryl)₂, -N(C₁₋₆Alkyl)(C₆₋₁₄Aryl),
-CH₃, -CHF₂, -CF₃, -C₂H₅, -C(CH₃)₂, -(CH₂)₂CH₃, -(CH₂)₃CH₃, -CH₂CH₂OH, -CH₂CH₂SH, -CH₂CH₂SCH₃, -CH₂CF₃, -CH₂CCl₃, -CH₂CHF₂, -CH₂CHCl₂, -CH₂CH₂F, -CH₂CH₂Cl, -CH₂CH₂Br, -Cylopropyl, -Cylopropylmethyl, -Cylobutyl, -Cyclobutylmethyl, -Cyclopentyl, -Cyclopentylmethyl, -Cylohexyl, -Cyclohexylmethyl,
-F, -Cl, -Br, -I, -CN, -NO₂, und -SCN.
sowie deren Solvate, einschließlich der pharmazeutisch akzeptablen Salze und Prodrug-Formulierungen und deren aktive Metabolite und gegebenenfalls deren Tautomere
zur Herstellung eines Arzneimittels zur Inhibierung von TNF-alpha.

9. Verwendung nach Anspruch 8 zur Behandlung von Rheumatoider Arthritis, Osteoarthritis, Osteoporosis, Asthma bronchiale, chronischer obstruktiver pulmonäre Erkrankung (COPD), Autoimmun-Erkrankungen wie z.B. Systemischer Lupus Erytematodes (SLE), Sklerodermie, Crohns Disease, Colitis ulcerosa, Multipler Sklerose, Guillain-Barré-Syndrom, Crohn's Disease, Colitis ulcerosa, Psoriasis, Graft-versus-Host-Disease, systemischem Alzheimer-Erkrankung, insulin-abhängiger Diabetes mellitus, Respiratorischem Distress-Syndrom beim Erwachsenen (ARDS), Sepsis, cerebrale Form der Malaria, Guillain-Barre-Syndrom, Graft-versus-Host-Disease (GvHD), multiples Organversagen nach Trauma, aktuter Glomerulonephritis, akute und chronische Schmerzen, Arteriosklerose, Herzinfarkt, Schlaganfall,, entzündlichen Dermatosen wie Psoriasis, atopische Dermatitis, Alopecie, akuter Meningitis, Vasculitis, Uveitis,, atopischer Dermatitis, cardiovaskuläre Erkrankungen, virale Erkrankungen und hier insbesondere retrovirale Erkrankungen wie z.B. das erworbene Immundefizienz Syndrom (AIDS) sowie Krebs, insbesondere Entartungen des blutbildenden Systems, neurodegenarativen Erkrankungen wie z.B. Mb. Alzheimer, Mb. Parkinson, Rhinitis allergica, allergische Konjunktivitis, Myastenia Gravis, und Sarkoidose.
